(19) Europäisches Patentamt
European Patent Office
Office européen des brevets

(11) Publication number: 0 228 416
B1

(12) # EUROPEAN PATENT SPECIFICATION

(45) Date of publication of the patent specification: **21.03.90**

(51) Int. Cl.[5]: **A 61 K 31/44,** A 61 K 31/505, A 61 K 31/435, A 61 K 31/47

(21) Application number: **86904018.8**

(22) Date of filing: **18.06.86**

(86) International application number: **PCT/US 86/01298**

(87) International publication number: **WO 86/07537 (31.12.86 Gazette 86/28)**

(54) **PHARMACEUTICAL COMPOSITIONS CONTAINING AZANAPHTHALENES.**

(30) Priority: **18.06.85 US 746149**
**12.12.85 US 808404**
**13.03.86 US 839308**
**12.06.86 US 871691**

(43) Date of publication of application: **15.07.87 Bulletin 87/29**

(45) Publication of the grant of the patent: **21.03.90 Bulletin 90/12**

(84) Designated Contracting States: **AT BE CH DE FR GB IT LI LU NL SE**

(56) References cited:
**EP-A-0 092 786**
**EP-A-0 127 135**
**EP-A-0 144 996**

**Chem. Abstracts vol. 88 no.15, 10 04 78. Columbus Ohio US. p 570, abstract 105298y**

**Goodman and Gilman "The Pharmacological Basis of herapeutics" 7th. ed. MacMillan,New York, pp 674-676**

(73) Proprietor: **SCHERING CORPORATION**
**2000 Galloping Hill Road**
**Kenilworth New Jersey 07033 (US)**

(72) Inventor: **SMITH, Sidney, Randall**
**700 Spring Avenue**
**Ridgewood, NJ 07450 (US)**
Inventor: **SIEGEL, Marvin, Ira**
**507 Maple Hill Drive**
**Woodbridge, NJ 07095 (US)**

(74) Representative: **von Kreisler, Alek, Dipl.-Chem.**
**Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).



LIBERGRAF, STOCKHOLM 1990

**Description**

The present invention relates to the use of azanaphthalenes for preparing a medicament and to pharmaceutical compositions containing the azanaphthalenes for suppressing the immune response. In particular it relates to the use of spiro azanaphthalene derivatives, naphthyridines and tricyclic quinoline, naphthyridine and pyridopyrazine derivatives.

The preparation of the spiro [cyclopentane]-quinolinedione is described in Chem. Pharm. Bull., 17, 1290 (1969). Several additional spiroquinoline diones are disclosed in Bull. Soc. Chim. Fr., 364 (1968). The references do not describe pharmaceutical uses for these compounds.

Japanese Patent 54152/83 discloses various naphthyridine derivatives which allegedly possess analgesic, anti-inflammatory, central nervous system depressant and diuretic effects. U.S. Patent 4 452 800 discloses various salts of 3-(n-butyl)-4-hydroxy-1-phenyl-1,8-naphthyridine-2-(1H)-one. U.S. Patent Nos. 4 492 702 and 4 551 463 disclose the use of various naphthyridine derivatives in treating allergic reactions and in treating and preventing ulcers in mammals. There is no indication in any of these references that such compounds may provide immunomodulating activity.

The preparation of the compound 2'-methyl-pyrano-5',6':3,4-(2-oxo-1,2-dihydroquinoline) and its N-phenyl derivative is described in Bull. Soc. Chim. Fr., pp. 364-9 (1968) (C.A. 68:114419c).

The present invention is drawn to the use of an azanaphtelene having the structural formula $I_a$, $I_b$ or $I_c$ or a solvate thereof for preparing a medicament and pharmaceutical compositions for suppressing the immune response, $I_a$ being:

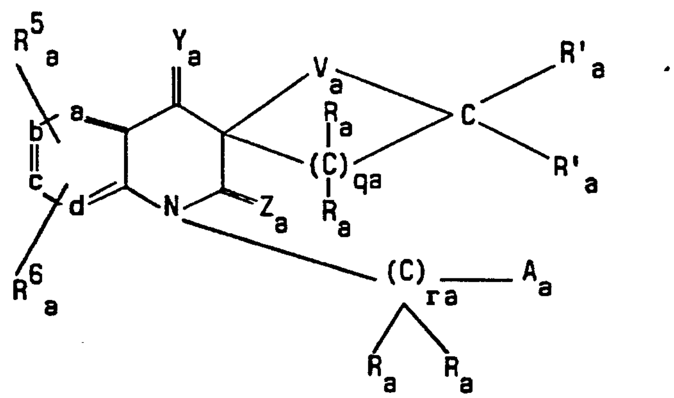

$I_a$

wherein:

two of the ring groups a, b, c and d may be CH or N and the remaining two groups represent CH;

$Y_a$ and $Z_a$ independently represent O or S;

$V_a$ represents O, $S(O)_{na}$, N-$R^8$a or $C(R_a)2$;

each $R_a$ independently represents hydrogen, alkyl having from 1 to 6 carbon atoms, $CH_2OH$, $COR^7{}_a$ (wherein $R^7{}_a$ represents hydrogen or alkyl having from 1 to 6 carbon atoms) or hydroxy, with the proviso that only one hydroxy group can be attached to one carbon atom;

each $R'_a$ independently is as defined for $R_a$ above, except that when $V_a$ represents O, S $(O)_{na}$ or N-$R^8{}_a$, $R'_a$ may not be hydroxy;

$R^8{}_a$ is hydrogen, alkyl having from 1 to 6 carbon atoms, carboxylic acyl having from 2 to 7 carbon atoms, alkylsulfonyl having from 1 to 6 carbon atoms, carboalkoxy having from 2 to 7 carbon atoms, $CONH_2$, phenyl or pyridyl of which the last two may be substituted with up to three substituents $Q_a$, whereby each $Q_a$ independently is hydroxy, alkyl having from 1 to 6 carbon atoms, halogen, nitro, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, $S(O)_{na}$-$R_a$ {wherein na is as defined above and $R^9{}_a$ is alkyl having from 1 to 6 carbon atoms}, $NHSO_2R^9{}_a$ {wherein $R^9{}_a$ is as defined above}, $NHSO_2CF_3$, $SO_2NH_2$, $COR^{10}{}_a$ {wherein $R^{10}{}_a$ is OH, $NH_2$ or $OR^9{}_a$ (wherein $R^9{}_a$ is as defined above)}, O-$B_a$-$COR^{10}{}_a$ {wherein $B_a$ is alkylene having from 1 to 4 carbon atoms and $R^{10}{}_a$ is as defined above}, or $NHCOR^{11}{}_a$ {wherein $R^{11}{}_a$ is hydrogen, alkyl having from 1 to 6 carbon atoms, alkoxy having from 1 to 6 carbon atoms, $COR^{12}{}_a$ (wherein $R^{12}{}_a$ is hydroxy or alkoxy having from 1 to 6 carbon atoms) or $NHR^{13}{}_a$ (wherein $R^{13}{}_a$ is hydrogen or alkyl having 1 to 6 carbon atoms)};

$R^5{}_a$ and $R^6{}_a$ may be the same or different and are hydrogen, alkyl having from 1 to 6 carbon atoms, halogen, nitro, alkoxy having from 1 to 6 carbon atoms, trifluoromethyl, alkylthio having 1 to 6 carbon atoms or cyano;

na is 0, 1 or 2;

ra is 0, 1 or 2;

qa is an integer of from 1 to 5; and

$A_a$ is phenyl, naphthyl, indenyl, indanyl, 2-,3- or 4-pyridyl, 2-, 4-, 5- or 6-pyrimidyl, 2- or 3-pyrazinyl, 2- or 3-furyl, 2- or 3-thienyl, 2-, 4- or 5-imidazolyl, 2-, 4- or 5-thiazolyl or 2-, 4- or 5-oxazolyl, any of which may be

substituted with up to three substituents $Q_a$ as defined herein above;
$I_b$ being:

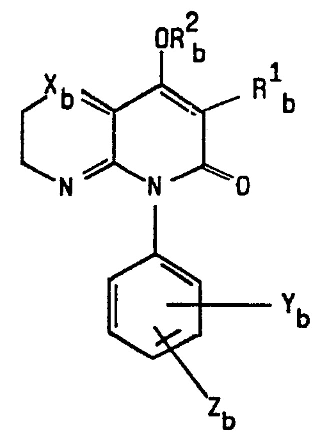

$I_b$

wherein $X_b$ is CH or N;

$Y_b$ is hydrogen, hydroxy, benzyloxy, amino, sulfamyl, halogen, nitro, alkyl having from 1 to 6 carbon atoms, alkoxy having from 1 to 6 carbon atoms, carboxylic acyl having from 2 to 6 carbon atoms, alkyl-S(O)$_{mb}$- having from 1 to 6 carbon atoms wherein mb is 0, 1 or 2, trifluoromethyl, trifluoromethylthio, or $COOA_b$ wherein $A_b$ is hydrogen, alkyl having from 1 to 6 carbon atoms or a cation derived from a pharmaceutically acceptable metal or an amine;

$Z_b$ is hydrogen, hydroxy, halogen, alkyl having from 1 to 6 carbon atoms, alkoxy having from 1 to 6 carbon atoms, hydroxyalkyl having from 1 to 6 carbon atoms, or carboxylic acyloxy having from 2 to 6 carbon atoms;

$R^1_b$ is alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 3- or 4-pyridyl, 2-,4- or 5-pyrimidyl, 2- or 3-thienyl, 2- or 3-furanyl, carboxylic acyl having from 2 to 6 carbon atoms or alkyl having from 1 to 10 carbon atoms which may be substituted with -COOH, hydroxy, halogen, alkoxy having from 1 to 6 carbon atoms, phenyl, 2-, 3- or 4-pyridyl, 2-, 4- or 5-pyrimidyl, 2- or 3-thienyl, 2- or 3-furanyl, carboxylic acyl having from 2 to 6 carbon atoms, cycloalkyl having from 3 to 7 carbon atoms or carboxylic acyloxy having from 1 to 6 carbon atoms;

$R^2_b$ is hydrogen, carboxylic acyl having from 1 to 6 carbon atoms, alkenyl having from 3 to 8 carbon atoms, alkynyl having from 3 to 8 carbon atoms, alkyl having from 1 to 6 carbon atoms, $R^3_bR^4_bN(CH_2)_{nb}$- (wherein $R^3_b$ and $R^4_b$ are hydrogen, alkyl having from 1 to 6 carbon atoms or may be joined to complete a piperidine, morpholine, piperazine or pyrrolidine ring and nb is an integer of from 2 to 6), hydroxyalkyl having from 2 to 6 carbon atoms, dihydroxyalkyl having from 2 to 6 carbon atoms, hydroxyalkoxyalkyl having from 2 to 8 carbon atoms, or a cation derived from a pharmaceutically acceptable metal or an amine;

$I_c$ being:

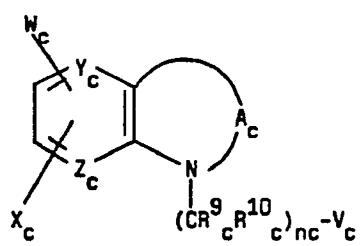

$I_c$

wherein:

$A_c$ is

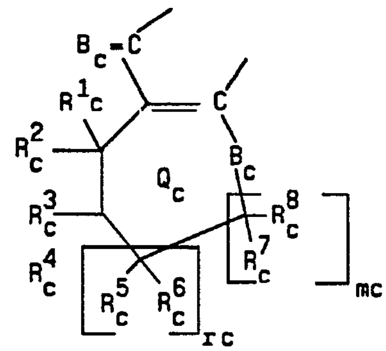

or

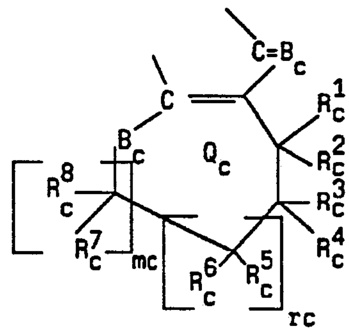

$A_c'$ $A_c''$

$B_c$ is independently oxygen or sulfur; $R^1{}_c$-$R^8{}_c$ may be the same or different and are hydrogen or alkyl having from 1 to 6 carbon atoms or two adjacent $R^1{}_c$-$R^8{}_c$ substituents may be combined to form an additional carbon to carbon bond;

rc and mc may be the same or different and are 0 or one;

the ring labeled $Q_c$ may optionally contain up to two additional double bonds;

nc is 0, 1 or 2;

$W_c$ and $X_c$ may be the same or different and are hydrogen, hydroxy, alkyl having from 1 to 6 carbon atoms, halogen, nitro, alkoxy having from 1 to 6 carbon atoms, tri-fluoromethyl, cyano, cycloalkyl having from 3 to 7 carbon atoms, alkenyloxy having from 3 to 6 carbon atoms, alkynyloxy having from 3 to 6 carbon atoms, $S(O)_{pc}$-$R^{11}{}_c$ {wherein pc is 0, 1 or 2 and $R^{11}{}_c$ is alkyl having from 1 to 6 carbon atoms}, $NHSO_2R^{11}{}_c$ {wherein $R^{11}{}_c$ is as defined above}, $NHSO_2CF_3$, $NHCOCF_3$, $SO_2NH_2$, $COR^{12}{}_c$ (wherein $R^{12}{}_c$ is OH, $NH_2$ or $OR^{11}{}_c$ (wherein $R^{11}{}_c$ is as defined above), O-$D_c$-$COR^{12}{}_c$ {wherein $D^c$ is alkylene having from 1 to 4 carbon atoms and $R^{12}{}_c$ is as defined above}, or $NHCOR^{13}{}_c$ {wherein $R^{13}{}_c$ is hydrogen, alkyl having from 1 to 6 carbon atoms, alkoxy having from 1 to 6 carbon atoms, $COR^{14}{}_c$ (wherein $R^{14}{}_c$ is hydroxy or alkoxy having from 1 to 6 carbon atoms) or $NHR^{15}{}_c$ (wherein $R^{15}{}_c$ is hydrogen or alkyl having from 1 to 6 carbon atoms)}, or phenoxy {wherein the benzene ring may be substituted with any of the other substituents $W_c$ and $X_c$};

$Y_c$ and $Z_c$ may be the same or different and are CH or N;

$V_c$ is phenyl, naphthyl, indenyl, indanyl, 2-, 3- or 4-pyridyl, 2-, 3- or 5-pyrimidinyl, 2- or 3-thienyl, 2- or 3-furyl or 2-, or 4- or 5-thiazolyl, any of which may be substituted with $W_c$ and $X_c$ as defined above; and $R^9{}_c$ and $R^{10}{}_c$ are independently hydrogen or alkyl having from 1 to 6 carbon atoms.

The compounds of formulae Ia, Ib and Ic can exist in unsolvated as well as solvated forms, including hydrated forms. In general, the solvated forms, with pharmaceutically acceptable solvents such as water and ethanol are equivalent to the unsolvated forms for purposes of the invention.

Certain compounds of the invention may exist in isomeric forms. The invention contemplates all such isomers both in pure form and in admixture, including racemic mixtures.

The azanaphthalenes defined above are known as possessing anti-allergy and/or anti-inflammatory activity. According to the present invention these compounds possess immunosuppressive activity, which was not to be expected on the basis of the prior art.

The compounds of formula Ia are disclosed in European published application No. 84 114 974.3 (European patent publication No. 0 144 996 A2), and are described as having anti-allergy and anti-inflammatory activities.

The compounds of formula Ib include those disclosed in U.S. Patent Nos. 4 452 800, 4 492 702 and 4 551 463 and the pharmaceutically acceptable metal or amino salts of the compound 3-(n-butyl )-4-hydroxy-1-phenyl-1,8-naphthyridine-2(1H)-one which is disclosed in Japanese patent public disclosure (Kokai ) 116 495/77, September 29, 1977, now Japanese Patent 54 152/83.

The compounds of formula Ic are disclosed in the European published patent Application No. 84 105 923.1 (publication number: 0 127 135 ). As disclosed in the European published application No. 84 105 923.1 (European patent publication No. 0 127 135 ), these compounds possess anti-allergy and anti-inflammatory activities.

A preferred subgenus of compounds of formula $I_a$ is that wherein $Y_a$ and $Z_a$ are both oxygen.

Preferably, a, b and c represent CH and d is either CH or N. A further preferred feature is that ra is zero, i.e. the group $A_a$ is directly attached to the ring-nitrogen atom. qa is preferably 2, 3 or 4, $V_a$ most preferably is $CH_2$ or O and $R^5{}_a$ and $R^6{}_a$ are both hydrogen.

Particularly preferred are compounds of the structural formula $II_a$:

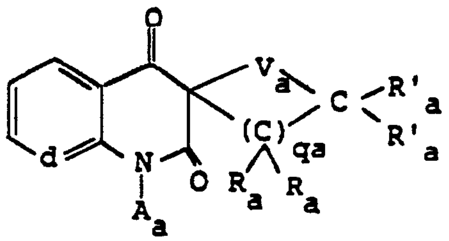

IIa

wherein d is CH or N, qa is 2, 3 or 4 and $A_a$, $V_a$, $R_a$ and $R'_a$ are as defined above.

When utilized herein and in the appended claims the below listed terms, unless specified otherwise, are defined as follows:

halogen – comprises fluorine, chlorine, bromine and iodine;

alkyl and alkoxy – comprises straight and branched carbon chains containing from 1 to 6 carbon atoms;

alkenyloxy – comprises straight and branched carbon chains containing from 3 to 6 carbon atoms and comprising a carbon to carbon double bond; and

alkynyloxy – comprises straight and branched carbon chains containing from 3 to 6 carbon atoms and comprising a carbon to carbon triple bond.

The compounds of the invention include a

$-(C)_{ra}$ and a $-(C)_{qa}$ – substituent wherein the $R_a$ groups may vary independently.

/ \ / \

$R_a$ $R_a$ $R_a$ $R_a$

Thus, for example, when ra or qa equals 2 the following patterns of substitution (wherein hydrogen and $CH_3$ are used to represent any substituents $R_a$) are contemplated: $-C(CH_3)_2CH_2-$, $-CH_2C(CH_3)_2-$, $-CH_2CH(CH_3)-$, $-CH(CH_3)CH_2-$ and $-(C(CH_3)H)_2-$. In addition when ra or qa equals 2, substituents such as $-C(CH_3)_2CH(C_2H_5)-$, $-CH(CH_3)CH(C_2H_5)$ are also contemplated.

It is obvious to one of ordinary skill in the art that due to problems of stability there are limitations involving the $R_a$ and $R'_a$ groups. One limitation is that neither $R_a$ can be a hydroxy group attached to the carbon alpha to the ring nitrogen atom. Another limitation is that the $R_a$ and $R'_a$ groups cannot both be hydroxy groups attached to the same carbon atoms.

Certain compounds of the invention may exist in isomeric forms. The invention contemplates all such isomers both in pure form and in admixture, including racemic mixtures. In the structural formulas $I_a$ and $II_a$ herein, when $V_a$ represents a hetero atom in the spiro ring, $V_a$ is attached directly to the spiro carbon atom, i.e., the carbon atom identified as number 3 in structural formula $I_a$.

The preferred compounds of formula $I_a$ summerized as follows:

compounds of formula Ia wherein $Y_a$ and $Z_a$ are both oxygen and/or
ra is zero and/or
$R^5_a$ and $R^6_a$ are both hydrogen and/or
qa is 2, 3 or 4 and/or
$R_a$ and $R'_a$ independently are hydrogen, methyl, ethyl, propyl, isopropyl, n-butyl or iso-butyl and/or
$A_a$ is phenyl or phenyl substituted with one or two substituents $Q_a$ and/or
$V_a$ is O, $S(O)_{na}$ or $N\text{-}R^8_a$ preferably O;
wherein all of a, b, c, and d are CH or
d is N and a, b and c are CH.

Representative compounds of formula $I_a$ are exemplified below in Table I:

**Table 1**

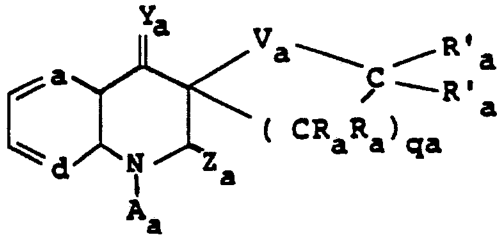

IIIa

| Compound No. | d | a | $A_a$ | $Y_a$ | $Z_a$ | $V_a$ | $-C(R'_a)_2(CR_aR_a)_{qa}-$ | m.p.(°C) |
|---|---|---|---|---|---|---|---|---|
| 1 | N | CH | phenyl | O | O | $CH_2$ | trimethylene | 174 – 178 |
| 2 | N | CH | 3-hydroxy phenyl | O | O | $CH_2$ | trimethylene | 218 – 220 |
| 3 | N | CH | 3-methoxy phenyl | O | O | $CH_2$ | trimethylene | 159 – 160.5 |
| 4 | CH | CH | phenyl | O | O | $CH_2$ | trimethylene | 168 – 168 |
| 5 | N | CH | 3,4-di-chloro phenyl | O | O | $CH_2$ | trimethylene | 143 – 145.5 |
| 6 | N | CH | 4-chloro phenyl | O | O | $CH_2$ | trimethylene | 168.5 – 172 |
| 7 | N | CH | 4-methyl phenyl | O | O | $CH_2$ | trimethylene | 177 – 178.5 |
| 8 | N | CH | 3-chloro phenyl | O | O | $CH_2$ | trimethylene | 165 – 167 |
| 9 | N | CH | 3-chloro phenyl | S | O | $CH_2$ | trimethylene | 168 – 170 |
| 10 | N | CH | phenyl | S | O | $CH_2$ | trimethylene | 188 – 189.5 |
| 11 | N | CH | 3-ethyloxalyl-amino phenyl | O | O | $CH_2$ | trimethylene | 158 – 160 |
| 12 | N | CH | 3-fomylamino-phenyl | O | O | $CH_2$ | trimethylene | 222 – 224 |
| 13 | N | CH | 3-aminophenyl | O | O | $CH_2$ | trimethylene | 200 – 202 |
| 14 | N | CH | 3-(ethyloxy-carbonyl-methoxy) phenyl | O | O | $CH_2$ | trimethylene | 103 – 105 |
| 15 | N | CH | phenyl | O | O | O | trimethylene | 241.5 – 243 (1/3 hydrate) |
| 16 | N | CH | phenyl | O | O | O | ethylene | 233 – 235.5 |
| 18 | N | CH | phenyl | O | O | S | trimethylene | |
| 19 | N | CH | 3,4-dichloro phenyl | O | O | O | tetramethylene | |
| 20 | N | CH | 3-chloro-phenyl | O | O | O | tetramethylene | 158.5 – 160 |
| 21 | N | CH | 4-chloro-phenyl | O | O | O | tetramethylene | 229- 231.5 (hemihydrate) |
| 22 | N | CH | 3-methoxy-phenyl | O | O | O | tetramethylene | 181 – 183 |
| 23 | N | CH | 4-methoxy phenyl | O | O | O | tetramethylene | |

Preferred compounds of formula $I_b$ are as follows:

The preferred value for $X_b$ is CH.

The preferred values for $Y_b$ are hydrogen, methoxy, trifluoromethyl, methylthio; the more preferred value is hydrogen.

The preferred values for $Z_b$ are hydrogen and methyl.

The preferred values for $R^1_b$ are n-alkyl having from 3 to 5 carbon atoms, alkenyl having from 3 to 4 carbon atoms, omega-hydroxyalkyl having 2 to 4 carbon atoms, and omega-carboxylicacyloxyalkyl having from 6 to 9 carbon atoms; the most preferred values are n-butyl, propen-2-yl, 2-hydroxyethyl, 3-hydroxypropyl and 4-propanoyloxybutyl.

The preferred values for $R^2_b$ are hydrogen, carboxylic acyl of from 2 to 4 carbon atoms, hydroxyalkyl of from 2 to 4 carbon atoms, $R^3_b(R^4_bN(CH_2)_{nb}$- (wherein $R^3_b$ and $R^4_b$ are each independently hydrogen or alkyl having from 1 to 6 carbon atoms and nb is an integer from 2 to 6 carbon atoms) and the cations derived from sodium, potassium, calcium, ethanolamine, N-methylglucamine, diethanolamine, ethylenediamine, tris-(hydroxymethyl)-aminomethane and lysine; the most preferred values are hydrogen, ethanoyl, propanoyl, 2-hydroxyethyl, and the cations derived from sodium, N-methylglucamine and lysine.

A preferred subgenus of compounds of formula $I_b$ are compounds of formula $II_b$:

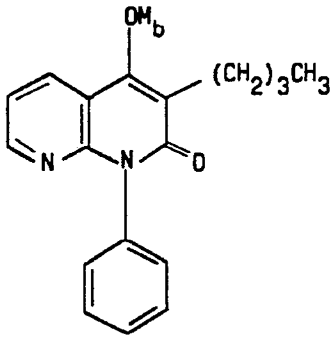

$II_b$

wherein $M_b$ is a cation derived from a pharmaceutically acceptable non-toxic metal or an amine. The preferred values for $M_b$ are the cations derived from sodium, potassium, calcium, ethanolamine, N-methylglucamine, diethanolamine, ethylenediamine, tris-(hydroxymethyl) aminomethane and lysine. The more preferred values for $M_b$ are the cations derived from sodium, N-methylglucamine and lysine.

Preferred compounds for use in the present invention include compounds having the formulae $III_b$-$IX_b$:

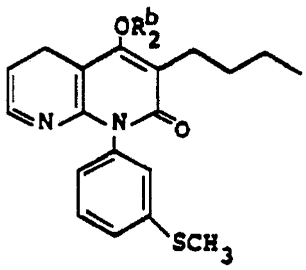

$III_b$

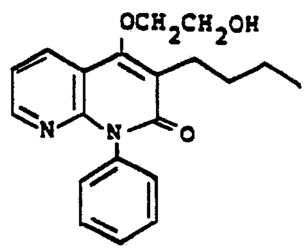

$IV_b$

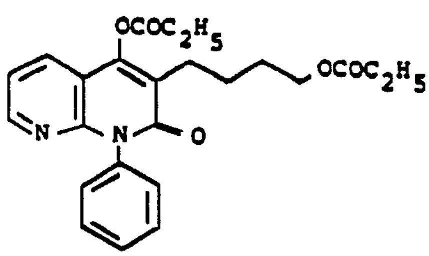

$V_b$

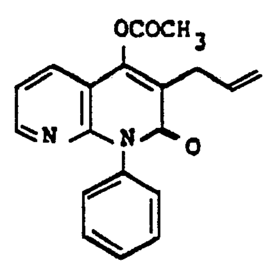

$VI_b$

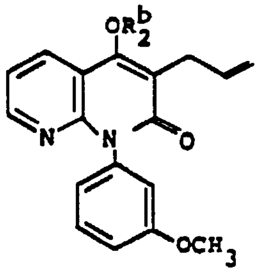

$VII_b$

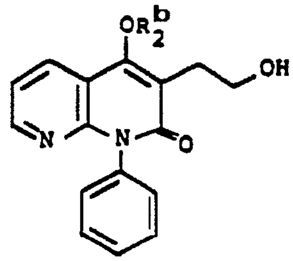

$VIII_b$

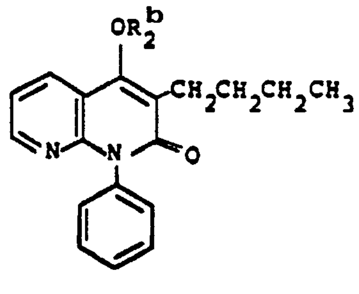

$IX_b$

wherein $R^2{}_b$ is hydrogen or the sodium cation.

The compounds of formula $I_b$ are substituted 1,8-naphthyridines and 1,5,8-azanaphthyridines and may exist as solvates, for example as hydrates. These compounds include salts formed at the 4-hydroxy group of 3-(n-butyl)-4-hydroxy-1-phenyl-1,8-naphthyridine-2(1H)-one.

The sodium and potassium salts of this invention can be readily prepared by reacting the appropriate compound, e.g., 3-(n-butyl)-4-hydroxy-1-phenyl-1,8-naphthyridine-2(1H)-one, with respectively, sodium hydroxide solution or potassium hydroxide solution.

The amine and amino acid salts can be prepared by reacting the appropriate amine or amino acid with the desired hydroxylated compound, e.g., 3-(n-butyl)-4-hydroxy-1-phenyl-1, 8-naphthyridine-2(1H)-one, in a compatible organic solvent, e.g. methanol.

The calcium salt can be prepared by reaction of the sodium salt with calcium chloride solution.

The aluminum, bismuth, chromium, copper, iron, magnesium, manganese and zinc salts of this invention can be prepared by methods known to those of skill in the art.

When utilized herein and in the appended claims the below listed terms, unless specified otherwise, are defined as follows:

halogen – fluorine, chlorine, bromine and iodine;

alkyl – straight and branched carbon chains containing from 1 to 10 carbon atoms;

hydroxyalkyl and dihydroxyalkyl having from 2 to 6 carbons – hydroxyalkyl and dihydroxyalkyl groups wherein the hydroxy group(s) is not substituted at the position alpha to the oxygen to which the $R^2{}_b$ group is attached.

$R^2{}_b$ is alkenyl and alkynyl having from 3 to 8 carbon atoms-alkenyl and alkynyl groups wherein the unsaturation is not at the position alpha to the oxygen to which the $R^2{}_b$ group is attached.

Pharmaceutically acceptable metal and amine – metals and amines that are generally recognized as being non toxic, such as sodium, potassium, calcium, aluminum, N-methylglucamine and lysine.

When used in the definition of compounds of formula Ic the below listed terms, unless specified otherwise, are defined as follows:

halogen – fluorine, chlorine, bromine and iodine;

alkyl and alkoxy – comprised of straight and branched carbon chains containing from 1 to 6 carbon atoms;

alkenyloxy – comprised of straight and branched carbon chains containing from 3 to 8 carbon atoms and comprising a carbon to carbon double bond; and

alkynyloxy – comprised of straight and branched carbon chains containing from 3 to 8 carbon atoms and comprising a carbon to carbon triple bond.

The compounds of the invention may possibly contain two different "$B_c$" substituents. It is intended that both may simultaneously be oxygen or sulfur, or that either may be oxygen or sulfur.

In certain compounds of the invention, the ring labeled $Q_c$, may contain up to two additional double bonds which double bonds are formed by the combination of two adjacent substituents, $R^1{}_c$-$R^8{}_c$. Thus, for example, when $Q_c$ is a 7 membered ring (rc and mc are both equal to one) it may contain 3 double bonds. When multiple double bonds are present, they will be non-cumulated double bonds.

The compounds of the invention are comprised of a $-(CR^9{}_cR^{10}{}_c)_{nc}-$ substituent wherein the $R^9{}_c$ and $R^{10}{}_c$ groups may vary independently. Thus, for example, when nc equals 2 the following patterns of substitution (wherein hydrogen and $CH_3$ are used to represent any substituent, $R^9{}_c$ or $R^{10}{}_c$) are contemplated: $-C(CH_3)_2CH_2-$, $-CH_2C(CH_3)_2-$, $-CH_2CH(CH_3)-$, $-CH(CH_3)CH_2-$ and $-(C(CH_3)H)_2-$. In addition when nc equals 2, substituents such as $-C(CH_3)_2CH(C_2H_5)-$, $-CH(CH_3)CH(C_2H_5)-$, $-CH(i\text{-}C_3H_7)CH(C_2H_5)-$ are also contemplated.

Subgenera of compounds of formula $I_c$ are
compounds wherein $B_c$ is oxygen;
compounds wherein $B_c$ is oxygen and $A_c$ is $A'_c$;
compounds wherein nc is zero and/or $Y_c$ is CH;
compounds having the structural formulas $II_c$, $III_c$, $IV_c$, $VI_c$, $VII_c$ or $VIII_c$

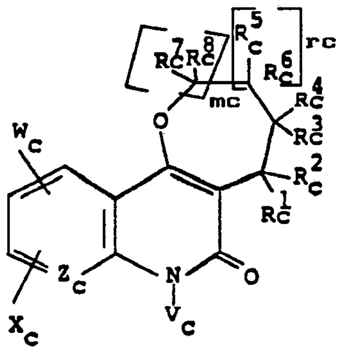

IIc

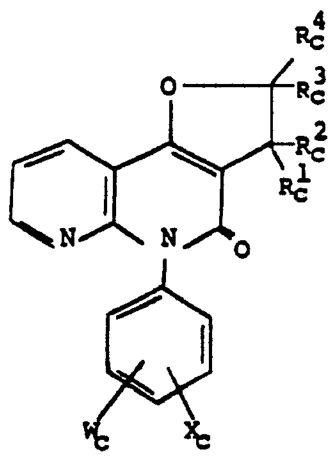

IIIc

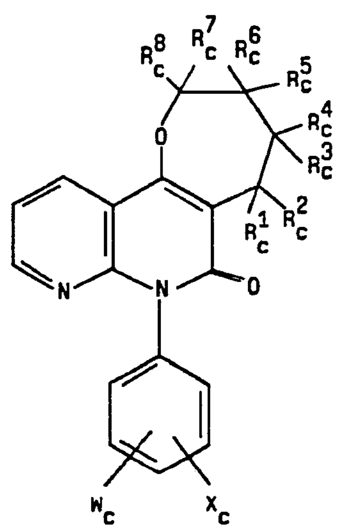

IVc

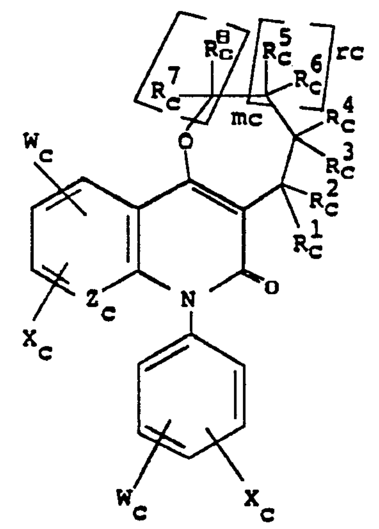

$VI_c$

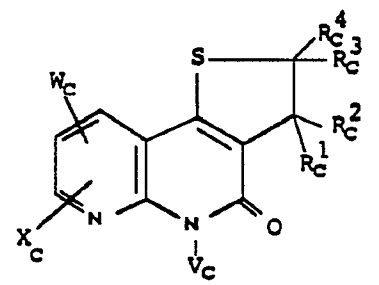

$VII_c$

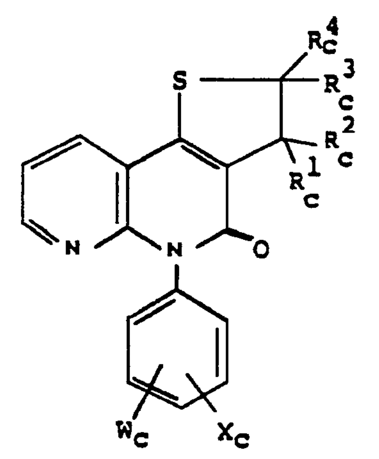

$VIII_c$

wherein $W_c$, $V_c$, $X_c$, $Z_c$, $R^1{}_c$-$R^8{}_c$, rc and mc are as defined herein;

compounds of formula $III_c$

wherein $W_c$, $X_c$, $R^1{}_c$, $R^2{}_c$, $R^3{}_c$, and $R^4{}_c$ are as defined above, wherein preferably $R^1{}_c$, $R^2{}_c$, $R^3{}_c$ and $R^4{}_c$ are hydrogen or methyl;

in particular compounds of formula $II_c$

wherein $W_c$, $X_c$, $Z_c$, $V_c$, $R^1{}_c$ to $R^8{}_c$, rc and mc are as defined above

wherein preferably
$Z_c$ is N and/or
rc and mc are both zero or one or
the sum of rc and mc is one and/or
$B_c$ is oxygen and/or
$V_c$ is of formula $IX_c$;

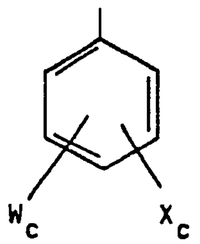

$IX_c$

compounds of formula $IV_c$

wherein $W_c$, $X_c$ and $R^1{}_c$-$R^8{}_c$ are as defined above, wherein preferably
$R^1{}_c$-$R^8{}_c$ are hydrogen or methyl, especially hydrogen,
or one of $R^1{}_c$-$R^8{}_c$ is methyl and the rest are hydrogen and/or
$W_c$ is 3-chloro and $X_c$ is hydrogen, chlorine or fluorine or
$W_c$ is 3-methoxy and $X_c$ is hydrogen or fluorine or
$W_c$ and $X_c$ are both hydrogen;

compounds of formula $VII_c$

wherein $W_c$, $X_c$, $V_c$, $R^1{}_c$, $R^2{}_c$, $R^3{}_c$ and $R^4{}_c$ are as defined above;
compounds of formula $VIII_c$

wherein $W_c$, $X_c$, $R^1{}_c$, $R^2{}_c$, $R^3{}_c$ and $R^4{}_c$ are as defined above, wherein preferably
$W_c$ is 3-chloro and $X_c$ is hydrogen, chlorine or fluorine or
$W_c$ is 3-methoxy and $X_c$ is hydrogen or fluorine or $W_c$ and $X_c$ are both hydrogen.

Representative compounds of formula $I_c$ are exemplified by the following compounds:

3,5-dihydro-5-phenyl-furo[3,2-c] [1,8]naphthyridin-4[2H]-one;
3,5-dihydro-5-phenyl-thieno[3,2-c] [1,8]-naphthyridin-4[2H]-one;
6-phenyl-2,3,4,6-tetrahydro-pyrano[3,2-c] [1,8]-naphthyridin-5-one;
2-methyl-3,5-dihydro-5-phenyl-furo[3,2-c] [1,8]-naphthyridin-4[2H]-one;
3,9-dihydro-9-phenyl-furo[2,3-b] [1,8]naphthyridin-4[2H]-one;
3,9-dihydro-9-(p-methylphenyl)-furo[2,3-b] [1,8]naphthyridin-4[2H]-one;
3,9-dihydro-2-methyl-9-phenyl-furo[2,3-b] [1,8]naphthyridin-4[2H]-one;
3,5-dihydro-5-(p-methylphenyl)-furo[3,2-c] [1,8]naphthyridin-4[2H]-one;
3,5-dihydro-5-(p-fluorophenyl)-furo[3,2-c] [1,8]naphthyridin-4[2H]-one;
3,5-dihydro-5-(m-methoxyphenyl)-furo[3,2-c] [1,8]naphthyridin-4[2H]-one;
3,5-dihydro-5-(m-methylthiophenyl)-furo[3,2-c] [1,8]-naphthyridin-4[2H]-one;
3,9-dihydro-9-(p-fluorophenyl)-furo[2,3-b] [1,8]naphthyridin-4[2H]-one;
3,9-dihydro-9-(m-methoxyphenyl)-furo[2,3-b] [1,8]naphthyridin-4[2H]-one;
3,9-dihydro-9-(m-methylthiophenyl)-furo[3,2-c] [1,8]-naphthyridin-4[2H]-one;
3,5-dihydro-5-(3,4-dichlorophenyl)-furo[3,2-c] [1,8]-naphthyridin-4[2H]-one;
3,5-dihydro-5-(3,4-dichlorophenyl)-2-methyl-furo[3,2-c] [1,8]-naphthyridin-4[2H]-one;
3,5-dihydro-5-(4-chlorophenyl)-furo[3,2-c] [1,8]-naphthyridin-4[2H]-one;
3,5-dihydro-5-(3-chlorophenyl)-furo[3,2-c] [1,8]-naphthyridin-4[2H]-one;
3,5-dihydro-5-(3-chlorophenyl)-2-methyl-furo[3,2-c] [1,8]-naphthyridin-4[2H]-one;
3,5-dihydro-5-(4-fluorophenyl)-2-methyl-furo[3,2-c] [1,8]-naphthyridin-4[2H]-one;
3,5-dihydro-5-(3-methoxyphenyl)-2-methyl-furo[3,2-c] [1,8]-naphthyridin-4[2H]-one;
3,5-dihydro-5-(3,5-dichlorophenyl)-furo[3,2-c] [1,8]-naphthyridin-4[2H]-one;
3,5-dihydro-5-(3,5-dichlorophenyl)-2-methyl-furo[3,2-c] [1,8]-naphthyridin-4[2H]-one;
3,5-dihydro-5-phenyl-2,2-dimethyl-furo[3,2-c] [1,8]-naphthyridin-4[2H]-one;
3,5-dihydro-5-(3-methylsulfonylaminophenyl)-2-methyl-furo[3,2-c] [1,8]-naphthyridin-4[2H]-one;
3,5-dihydro-5-(3-methylsulfonylaminophenyl)-furo [3,2-c] [1,8]-naphthyridin-4[2H]-one;

3,9-dihydro-9-(3,4-dichlorophenyl)-furo[2,3-b] [1,8]-naphthyridin-4[2H]-one;
3,9-dihydro-9-(4-chlorophenyl)-furo[2,3-b] [1,8]-naphthyridin-4[2H]-one;
3,9-dihydro-9-(3-chlorophenyl)-furo[2,3-b] [1,8]-naphthyridin-4[2H]-one;
3,9-dihydro-9-(3-chlorophenyl)-2-methyl-furo[2,3-b] [1,8]-naphthyridin-4[2H]-one;
3,9-dihydro-9-(4-fluorophenyl)-2-methyl-furo[2,3-b][1,8]-naphthyridin-4[2H]-one;.
3,9-dihydro-9-(3-methoxyphenyl)-2-methyl-furo [2,3-b] [1,8]-naphthyridin-4[2H]-one;
3,9-dihydro-9-(3, 5-dichlorophenyl)-furo[2,3-b] [1,8]-naphthyridin-4[2H]-one;
3,9-dihydro-9-(3,5-dichlorophenyl)-2-methyl-furo [2,3-b] [1,8]-naphthyridin-4[2H]-one;
3,9-dihydro-9-(3-methylsulfonylaminophenyl)-2-methyl-furo [2,3-b] [1,8]-naphthyridin-4[2H]-one;
6-(4-chlorophenyl)-2,3,4,6-tetrahydro-5H-pyrano[3,2-c]-[1,8]naphthyridin-5-one;
6-(3,4-dichlorophenyl)-2,3,4,6-tetrahydro-5H-pyrano [3,2-c] [1,8]naphthyridin-5-one;
6-(4-methoxyphenyl)-2,3,4,-tetrahydro-5H-pyrano [3,2-c] [1,8]naphthyridin-5-one;
6-(4-methylphenyl)-2,3,4,6-tetrahydro-5H-pyrano [3,2-c] [1,8]naphthyridin-5-one;
10-(3,4-dichlorophenyl)-2,3,4,10-tetrahydro-5H-pyrano [2,3-b] [1,8]naphthyridin-5-one;
10-(4-methoxyphenyl)-2,3,4,10-tetrahydro-5H-pyrano-[2,3-b] [1,8]naphthyridin-5-one;
10-(4-chlorophenyl)-2,3,4,10-tetrahydro-5H-pyrano-(2,3-b] [1,8]naphthyridin-5-one;
10-(4-methylphenyl)-2,3,4,10-tetrahydro-5H-pyrano-(2,3-b] [1,8]naphthyridin-5-one;
10-phenyl-2,3,4,10-tetrahydro-5H-pyrano-[2,3-b] [1,8]-naphthyridin-5-one;
7-phenyl-3,4,5,7-tetrahydro-oxepino[3,2-c] [1,8]-naphthyridin-6[2H]-one;
7-(4-chlorophenyl)-3,4,5,7-tetrahydro-oxepino-[3,2-c] [1,8]-naphthyridin-6[2H]-one;
7-(3-chlorophenyl)-3,4,5,7-tetrahydro-oxepino-[3,2-c] [1,8]-naphthyridin-6[2H]-one;
7-(3-methoxyphenyl)-3,4,5,7-tetrahydro-oxepino-[3,2-c] [1,8]-naphthyridin-6[2H]-one; and
7-(3-hydroxyphenyl)-3,4,5,7-tetrahydro-oxepino-[3,2-c] [1,8]-naphthyridin-6[2H]-one.

The compounds are useful in the treatment of autoimmune and other immunological diseases including graft rejection in which T cell proliferation is a contributing factor to the pathogenesis of disease. The effectiveness of these compounds as immunosuppressing agents may be demonstrated by the following tests which involve the inhibition of T cell functions using these compounds.

**Graft VS. Host Reaction (GVHR)**

To induce a GVHR, C57 B1/6XA/J(B6AF1) male mice were injected intravenously with parental (C57B1/6J) spleen and lymph node cells. The test compound was then administered orally for 12 days beginning on the day prior to the cell transfer. On the day following the last treatment, the animals were sacrificed, and their spleens were excised and weighed. The enlargement of the spleen of the host is a result of a GVHR. To some extent it is the host's own cells which infiltrate and enlarge the spleen although they do this because of the presence of graft cells reacting against the host. The amount of spleen enlargement, splenomegaly, is taken as a measure of the severity of the GVHR.

In carrying out the GVHR the animal in the experimental group is injected with parental cells, cells of the same species but of different genotype, which cause a weight increase of the spleen. The animal in the control group is injected with syngeneic cells, genetically identical cells which do not cause a weight increase of the spleen. The effectiveness of the test compound administered to the mice in the experimental group is measured by comparing the spleen weight of the untreated and treated GVH animal with that of the syngeneic control.

$ED_{30}$: dosage [mg/kg] of test compound administered daily yielding a 30 % decrease of spleen weight as compared to the untreated animals.

| Test Compuond | $ED_{30}$ [mg/kg] |
|---|---|
| (R,S)-1-Phenyl-3',4',5',6'-Tetrahydro-Spiro-[1,8-Naphthyridine-3,2'-[2H]-Pyran]-2, 4-Dione 1/4 Hydrate | 100 |
| 1'-(3-Chlorophenyl)-Spiro[Cyclopentane-1,3'-[1,8] Naphthyridine]-2',4'-(1'H)-Dione | 100 |
| 4-Acetoxy-1-Phenyl-3-(2-Propenyl)-1,8-Naphthyridin-2(1H)-One | 25 |

**Splenic Atrophy**

The immunosuppressive activity of the compounds may also be shown by a decrease in spleen weight after dosing $BDF_1$ mice orally with the drug for seven (7) consecutive days. The mice are sacrificed on the eighth day. The percent decrease in spleen weight is measured for each dosage level. In this procedure 1'-(3-chlorophenyl)-spiro-[cyclopentane-1,3'-[1,8]-naphthyridine]-2',4'-(1H)-dione provided a 30 % spleen weight decrease at a dosage level of 100 mg/kg.

**Graft VS. Host Reaction (GVHR)**

To induce a GVHR, C57 B1/6XA/J(86AF1) male mice were injected intraperitoneally with parental (C57B1/6J) spleen cells. 3-(n-Butyl)-4-hydroxy-1-phenyl-1,8-naphthyridine-2(1H)-one sodium salt (test drug) was then administered orally for 8 days beginning on the day of cell transfer. On the day following the last treatment, body weights were obtained, the animals were sacrificed, and their spleens were excised and weighed. The enlargement of the spleen of the host is a result of a GVHR. To some extent it is the host's own cells which infiltrate and enlarge the spleen although they do this because of the presence of graft cells reacting against the host. The amount of spleen enlargement, splenomegaly, is taken as a measure of the severity of the GVHR.

The GVHR is expressed below as the spleen index, the ratio of the weight of the spleen to the total weight of the animal in the experimental group compared with the same ratio in the control group.

$$\text{Spleen index} = \frac{\text{weight of experimental spleen/total body weight}}{\text{weight of control spleen/total body weight}}$$

In carrying out the GVHR the animal in the experimental group is injected with parental cells, cells of the same species but of different genotype, which cause a weight increase of the spleen. The animal in the control group is injected with syngeneic cells, genetically identical cells, which do not cause a weight increase of the spleen. The effectiveness of the sodium salt administered to the mice in the experimental group is measured against a spleen index scale. The scale goes from a spleen index of 1.0 for complete immunosuppression to a spleen index of 2.6 for a lack of immunosuppression.

The sodium salt at 100 mg/kg gave a spleen index of 1.6.

According to the same test procedure BDFI mice were injected intravenously with 1 X $10^8$ $C_{57}$ B1/6 spleen and lymph node cells and the test drug was administered for 10 days. The following results were obtained:

**Test Drug**

| | Dose (mg/kg) | Per Cent Inhibition Spleen Enlargement | Spleen Index |
|---|---|---|---|
| 3-(n-butyl)-4-hydroxy-1-phenyl-1,8-naphthyridine-2(1H)-one | 100 | 71 | 71 |
| 3-(n-butyl)-4[2-(dimethylamino) ethoxy]-1-phenyl-1,8-naphthyridine -2(1H)-one hydrochloride | 50 | 54 | 43 |
| 3-(n-butyl)-1-(2,4-difluorophenyl)-4-hydroxy-1,8-naphthyridin-2(1H)-one | 100 | 11 | 10 |

The following result where obtained when a modification of the above test procedure was used, wherein BDFI mice were injected intravenously with 1 X $10^8$ $C_{57}$ B1/6 spleen cells and the mice were pretreated daily with drug for 7 days prior to and following the injection of parental spleen cells (total of 16 days):

**Test Drug**

| | Dose (mg/kg, p.o.) | Per Cent Inhibition Spleen Enlargement | Spleen Index |
|---|---|---|---|
| 3-(n-butyl)-4-hydroxy-1-phenyl-1,8-naphthyridine-2(1H)-one | 100 | 100 | 80 |
| 3-(n-butyl)-4[2-(dimethylamino) ethoxy]phenyl-1,8-naphthyridine -2(1H)-one hydrochloride | 50 | 52 | 19 |
| 3-(n-butyl)-1-(2,4-difluorophenyl)-4-hydroxy-1,8-naphthyridin-2(1H)-one | 100 | 37 | 37 |
| 7-(n-butyl)-8-hydroxy-5-phenyl-pyrido [2,3-b]pyrarin-6(5H)-one | 100 | 75 | 81 |

**T Cell Mitogenic Responsiveness**

Spleen cells were obtained from six to eight week old C57 B1/6J male mice. One million viable spleen cells were cultured in triplicate in microtest II plates in the presence of 1 µg concanavalin A (Con A) or 0.25 µg phytophemagglutinin (PHA) for 72 hr at 37°C . The total volume was 0.2 ml. One microcurie $^3$H-Thymidine (specific activity, 2.0 Ci/mmole) was added for the last 16 hr of incubation. The cells were harvested and processed on a mash II harvester. A stock solution of 1 x $10^{-2}$M of the test drug (inhibitor) was prepared in distilled water and then diluted with medium to the appropriate concentration. The test drugs at concentrations of 1,10 and 100 µM inhibited the proliferative responses of unfractionated murine splenocytes to the T cell mitogens, Con A and PHA as shown in the following table:

| Mitogen | Inhibitor | Per Cent Control Proliferative Response Inhibitor Concentration (µM) | | |
|---|---|---|---|---|
| | | 1 | 10 | 100 |
| Concanavalin A (Con A) | 3-(n-butyl)-4-hydroxy-1-phenyl-1,8-naphthyridin-2(1H)-one | 74 | 71 | 70 |
| | 3-(3-hydroxy-n-butyl)-4-hydroxy-1-phenyl-1,8-naphthyridin-2(1H)-one | 96 | 78 | 59 |
| | 4-hydroxy-1-phenyl-3-[2-(2-pyridinyl)ethyl]-1,8-naphthyridin-2(1H)-one | 89 | 66 | 47 |
| Phytohemagglutin (PHA) | 3-(n-butyl)-4-hydroxy-1-phenyl-1,8-naphthyridin-2(1H)-one | 80 | 49 | 31 |
| | 3-(3-hydroxy-n-butyl)-4-hydroxy-1-phenyl-1,8-naphthyridin-2(1H)-one | 103 | 102 | 62 |
| | 4-hydroxy-1-phenyl-3-[2-(2-pyridinyl)ethyl]-1,8-naphthyridin-2(1H)-one | 112 | 100 | 32 |

The immuno suppressive activity of the compounds of formula $I_c$ is shown by the following test results:

**Graft VS. Host Reaction (GVHR)**

To induce a GVHR, C57 B1/6XA/J(B6AF1) male mice were injected intravenously with parental (C57B1/6J) spleen and lymph node cells. The compound 10-(4-chlorophenyl)-2,3,4,10-tetrahydro-5H-pyrano[2, 3-b] [1,8] naphthyridin-5-one (test drug) was then administered orally for 10 days beginning on the day prior to the cell transfer. On the day following the last treatment, the animals were sacrificed, and their spleens were excised and weighed. The enlargement of the spleen of the host is a result of a GVHR. To some extent it is the host's own cells which infiltrate and enlarge the spleen although they do this because of the presence of graft cells reacting against the host. The amount of spleen enlargement, splenomegaly, is taken as a measure of the severity of the GVHR.

In carrying out the GVHR the animal in the experimental group is injected with parental cells, cells of the same species but of different genotype, which cause a weight increase of the spleen. The animal in the control group is injected with syngeneic cells, genetically identical cells which do not cause a weight increase of the spleen. The effectiveness of the test drug administered to the mice in the experimental group is measured by comparing the spleen weight of the untreated and treated GVH animal with that of the syngeneic control. The test drug reduced spleen weight by 8 % as compared to the untreated animals at a dose of 100 mg/kg.

**Anti-Sheep Red Blood Cell Response**

The immunosuppressive activity of the compounds of formula $I_c$ may also be shown by the inhibition of the secretion of IgM cells in mice immunized with sheep erythrocytes. In particular, $BDF_1$ mice are immunized intravenously with 1 x $10^8$ sheep erythrocytes on day zero. Treatment with the test drug (oral administration) is initiated the day prior to the immunization and is continued through day three. On day four the number of IgM secreting cells in the spleens of the treated mice are assessed by the Jerne Plaque technique, with the results expressed as a percent inhibition in comparison to untreated controls. 10-(4-chlorophenyl)-2,3,4,10-tetrahydro-5H-pyrano [2,3-b] [1,8]naphthyridin-5-one at 100 mg/kg per day and 9-(3-chlorophenyl)-3,9-dihydro-2-methyl-furo [2,3-b] [1,8]naphthyridin-4[2H]-one at 100 mg/kg per day provided about 10 % and about 27 -- 36 % inhibition, respectively, in this test procedure.

As noted, European patent publication No. 0 144 966 A2 discloses that the subject compounds (formula $I_a$) possess anti-allergy and anti-inflammatory activities. For example, 1-phenyl-3',4',5',6'-tetrahydro-spiro-[1,8]-naphthyridine-3,2'-[2H] pyran-2,4-dione 1/4 hydrate has an $ED_{50}$ value of about 2 mg/kg p.o. in tests measuring the inhibition of anaphylactic bronchospasm in sensitized guinea pigs having antigen-induced bronchoconstriction and an $ED_{50}$ value of about 19 mg/kg p.o. in tests measuring the reverse passive Arthus reaction in the pleural cavity of rats (as described by Myers et al., *Inflammation*, Vol. 9, No. 1, 1985, pp. 91 – 98). This compound has an $ED_{30}$ value of about 100 mg/kg in the GVHR test as described above. As noted, European patent publication No. 0 127 135 discloses that the subject compounds possess anti-allergy and anti-inflammatory activities. For example, 10-(4-chlorophenyl)-2,3,4,10-tetrahydro-5.H-pyrano [2,3-b] [1,8]naphthyridin-5-one at a concentration of about 10 μM provided about 57 % inhibition of SRS-A release in the procedure described in Kreutner et al., *European Journal of Pharmacology*, Vol. 111, 1985, pp 1 – 8. This compound has an $ED_{30}$ value of about 6 mg/kg p.o. in tests measuring the reverse passive Arthus reaction in the pleural cavity of rats (as described by Myers et al., *Inflammation*, Vol. 9, No. 1, 1985, pp. 91 – 98) . These results indicate that an immunosuppressive effective dose for the compounds formulae $I_a$, $I_b$, and $I_c$ is several times their anti-inflammatory and anti-allergy effective doses.

The compounds of formulae $I_a$, $I_b$ and $I_c$ are orally and parenterally effective in the treatment of autoimmune and other immunological disease including graft rejection at a dosage range of 0.1 to 250 mg/kg of body weight per day, preferably 0.1 to 150 mg/kg or 25 to 150 mg/kg.

In particluar, the usual dosage range for the compounds of formula $I_b$ in a 70 kg mammal is an oral dose of about 5 to 250 mg/kg, preferably 25 to 150 mg/kg, in 3 or 4 divided doses;

the usual dosage range for the compounds of formula $I_a$ in a 70 kg mammal is an *oral* dose of about .1 to 250 mg/kg, preferably .1 to 150 mg/kg, in 3 or 4 divided doses per day;

the usual dosage range for the compounds of formulae $I_c$ in a 70 kg mammal is a dose of about .1 to 250 mg/kg, preferably .1 to 150 mg/kg per day. The compounds may be administered by conventional routes, e.g., orally, subcutaneously, intramuscularly, etc. Orally the compound may be administered in 3 or 4 divided doses per day. Of course, the dose will be regulated according to the potency of compound employed, the immunological disease being treated, and the judgment of the attending clinician depending on factors such as the degree and the severity of the disease state and age and general condition of the patient being treated.

To treat immunological diseases, the active compounds of formulae $I_a$, $I_b$ and $I_c$ can be administered in unit dosage forms such as tablets, capsules, pills, powders, granules, sterile parenteral solutions or suspensions, suppositories and transdermal compositions. Such dosage forms are prepared according to standard techniques well known in the art.

For preparing pharmaceutical compositions from the compounds described by this invention, inert, pharmaceutically acceptable carriers can be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid-carriers are magnesium carbonate, magnesium strearate, talc, sugar, lactose, pectin, dextrin, starch, gelatin, tragacanth, methylcellulose, sodium carboxymethylcellulose, a low melting wax and cocoa butter. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution or suspension in aqueous polyethylene glycol solution. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the finely divided active component in water with viscous material, i.e., natural or synthetic gums, resins,

methylcellulose, sodium carboxymethylcellulose and other well-known suspending agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in additions to the active material, flavorants, colorants, stabilizers, buffers, artificial and natural sweeteners, dispersants, thickeners and solubilizing agents. The solvent utilized for preparing the liquid form preparation may be water, isotonic water, ethanol, glycerine and propylene glycol as well as mixtures thereof. Naturally, the solvent utilized will be chosen with regard to the route of administration, for example, liquid preparations containing large amounts of ethanol are not suitable for parenteral use.

The composition of the invention may also be deliverable transdermally. The transdermal compositions can take the form of creams, lotions and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be appropriate number of any of these in packaged form. The quantity of active compound in a unit dose of preparation may be varied or adjusted from 1 mg to 1000 mg according to the particular application and the potency of the active ingredient. The compositions can, if desired, also contain other therapeutic agents.

The dosages may be varied depending upon the requirements of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following examples are intended to illustrate, but not to limit, the present invention.

The term "Compound A", "Compound B" and "Compound C" refers to any one of the compounds of formula $I_a$ (Compound A), formula $I_b$ (Compound B) and formula $I_c$ (Compound C):

Compound A: 1'-phenyl-3',4',5',6'-tetrahydro-spiro-[1,8] naphthyridine]-3',2'-[2H-pyran]-2,4-dione 1/4 hydrate,
Compound B: 3-(n-butyl)-4-hydroxy-1-(3-methylthiophenyl)-1,8-naphthyridin-2-(1H)-one;
3-(n-butyl)-4-hydroxy-1-phenyl-1,8-naphthyridin-2(1H)-one;
4-acetoxy-1-phenyl-3-(2-propenyl)-1,8-naphthyridin-2-(1H)-one;
4-hydroxy-1-phenyl-3-(2-propenyl)-1,8-naphthyridin-2-(1H)-one;
4-hydroxy-1-(3-methoxyphenyl)-3-(2-propenyl)-1,8-naphthyridin-2(1H)-one;
1-phenyl-4-propionyloxy-3-(4-propionyloxybutyl)-1,8,-naphthyridin-2(1H)-one;
3-(2-hydroxyethyl)-4-hydroxy-1-phenyl-1,8-naphthyridin-2(1H)-one;
3-(n-butyl)-4-(2-hydroxyethoxy)-1-phenyl-1,8-naphthyridin-2(1H)-one;
4-hydroxy-1-phenyl-3-(2-pyridyl)-1,8-naphthyridin-2(1H)-one;
and 3-(n-butyl)-4-[2-(2-hydroxyethyoxy)ethoxy]-1-phenyl-1,8-naphthyridin-2(1H)-one,
Compound C: 10-(4-chlorophenyl)-2,3,4,10-tetrahydro-5H-pyrano[2,3-b] [1,8]-naphthyridin-5-one.

It is contemplated, however, that this compound may be replaced by equally effective quantities of other compounds of formulae $I_a$, $I_b$ and $I_c$ as defined above.

**Example 1**

**Tablets**

| No. | Ingredients | mg/tablet | mg/tablet |
|---|---|---|---|
| 1 | Compound A, B or C | 100 | 500 |
| 2 | Lactose USP | 122 | 113 |
| 3 | Corn Starch, Food Grade, as a 10 % paste in Purified Water | 30 | 40 |
| 4 | Corn Starch, Food Grade | 45 | 40 |
| 5 | Magnesium Strearate | 3 | 7 |
| | Total | 300 | 700 |

**Method of Manufacture**

Mix Item Nos. 1 and 2 in a suitable mixture for 10 – 15 minutes. Granulate the mixture with Item No. 3. Mill the damp granules through a coarse screen (e.g., 6.35mm (1/4") if needed. Dry the damp granules. Screen the dried granules if needed and mix with the Items No. 4 and mix for 10 – 15 minutes. Add Item No. 5 and mix for 1 – 3 minutes. Compress the mixture to appropriate the size and weight on a suitable tablet machine.

**Example 2**

**Capsules**

| No. | Ingredient | mg/capsule | mg/capsule |
|---|---|---|---|
| 1 | Compound A, B or C | 100 | 500 |
| 2 | Lactose USP | 106 | 123 |
| 3 | Corn Starch, Food Grade | 40 | 70 |
| 4 | Magnesium Strearate NF | 4 | 7 |
| | Total | 250 | 700 |

**Method of Manufacture**

Mix Item Nos. 1, 2 and 3 in a suitable blender for 10 – 15 minutes. Add Item No. 4 and mix for 1 – 3 minutes. Fill the mixture into suitable two-piece hard gelatin capsules on a suitable encapsulating machine.

**Example 3**

**Parenteral**

| Ingredient | mg/vial | mg/vial |
|---|---|---|
| Compound A or B Sterile Powder | 100 | 500 |

Add sterile water for injection or bacteriostatic water for injection for reconstruction.

**Example 4**

**Injectable**

| No. | Ingredient | mg/vial | mg/vial |
|---|---|---|---|
| 1. | Compound A or B | 100 | 500 |
| 2. | Methylparaben | 1.8 | 1.8 |
| 3. | Propylparaben | 0.2 | 0.2 |
| 4. | Sodium Bisulfilte | 3.2 | 3.2 |
| 5. | Disodium Edetate | 0.1 | 0.1 |
| 6. | Sodium Sulfate | 2.6 | 2.6 |
| 7. | Water for Injection q.s. ad | 1.0ml | 1.0ml |

**Method for Manufacture**

1. Dissolve parabens in a portion (85 %) of the final volume) of the water for injection at 65 – 70°C.
2. Cool to 25 – 35°C. Charge and dissolve the sodium bisulfite, disodium edetate and sodium sulfate.
3. Charge and dissolve drug.
4. Bring the solution to final volume by added water for injection.
5. Filter the solution through 0.22 membrane and fill into appropriate containers.
6. Terminally sterilize the units by autoclaving.

**Claims**

1. Use of an azanaphthalene having the structural formula $I_a$, $I_b$ or $I_c$

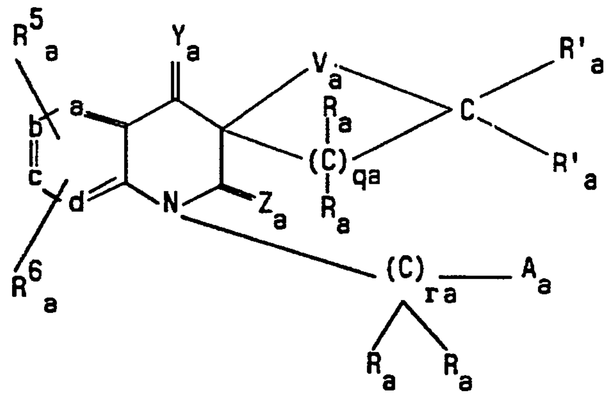

$I_a$

or a solvate thereof, wherein:

two of the ring groups a, b, c and d may be CH or N and the remaining two groups represent CH;

$Y_a$ and $Z_a$ independently represent O or S;

$V_a$ represents O, $S(O)_{na}$, N-$R^8_a$ or 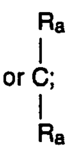

each $R_a$ independently represents hydrogen, alkyl having from 1 to 6 carbon atoms, $CH_2OH$, $COR^7_a$ (wherein $R^7_a$ represents hydrogen or alkyl having from 1 to 6 carbon atoms) or hydroxy, with the proviso that only one hydroxy group can be attached to one carbon atom;

each $R'_a$ independently is as defined for $R_a$ above, except that when $V_a$ represents O, S $(O)_{na}$ or N-$R^8_a$, $R'_a$ may not be hydroxy;

$R^8_a$ is hydrogen, alkyl having from 1 to 6 carbon atoms, carboxylic acyl having from 2 to 7 carbon atoms, alkylsulfonyl having from 1 to 6 carbon atoms, carboalkoxy having from 2 to 7 carbon atoms, $CONH_2$, phenyl or pyridyl of which the last two may be substituted with up to three substituents $Q_a$, whereby each $Q_a$ independently is hydroxy, alkyl having from 1 to 6 carbon atoms, halogen, nitro, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, $S(O)_{na}$-$R^9_a$ {wherein na is as defined herein and $R^9_a$ is alkyl having from 1 to 6 carbon atoms}, $NHSO_2R^9_a$ (wherein $R^9_a$ is as defined above), $NHSO_2CF_3$, $SO_2NH_2$, $COR^{10}_a$ {wherein $R^{10}_a$ is OH, $NH_2$ or $OR^9_a$ (wherein $R^9_a$ is as defined above}, O-$B_a$-$COR^{10}_a$ {wherein $B_a$ is alkylene having from 1 to 4 carbon atoms and $R^{10}_a$ is as defined above}, or $NHCOR^{11}_a$ {wherein $R^{11}_a$ is hydrogen, alkyl having from 1 to 6 carbon atoms, alkoxy having from 1 to 6 carbon atoms, $COR^{12}_a$ (wherein $R^{12}_a$ is hydroxy or alkoxy having from 1 to 6 carbon atoms) or $NHR^{13}_a$ (wherein $R^{13}_a$ is hydrogen or alkyl having 1 to 6 carbon atoms)};

$R^5_a$ and $R^6_a$ may be the same or different and are hydrogen, alkyl having from 1 to 6 carbon atoms, halogen, nitro, alkoxy having from 1 to 6 carbon atoms, trifluoromethyl, alkylthio having 1 to 6 carbon atoms or cyano;

na is 0, 1 or 2;

ra is 0, 1 or 2;

qa is an integer of from 1 to 5; and

$A_a$ is phenyl, naphthyl, indenyl, indanyl, 2-, 3- or 4-pyridyl, 2-, 4-, 5- or 6-pyrimidyl, 2- or 3-pyrazinyl, 2- or 3-furyl, 2- or 3-thienyl, 2-, 4- or 5-imidazolyl, 2-, 4- or 5-thiazolyl or 2-, 4- or 5-oxazolyl, any of which may be substituted with up to three substituents $Q_a$ as defined herein above;

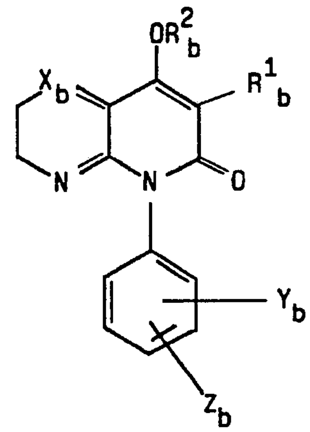

$I_b$

or a solvate thereof, wherein:

$X_b$ is CH or N;

$Y_b$ is hydrogen, hydroxy, benzyloxy, amino, sulfamyl, halogen, nitro, alkyl having from 1 to 6 carbon atoms, alkoxy having from 1 to 6 carbon atoms, carboxylic acyl having from 2 to 6 carbon atoms, alkyl-$S(O)_{mb}$- having from 1 to 6 carbon atoms wherein mb is 0, 1 or 2, trifluormethyl, trifluormethylthio, or $COOA_b$ wherein $A_b$ is hydrogen, alkyl having from 1 to 6 carbon atoms or a cation derived from a pharmaceutically acceptable metal or an amine;

$Z_b$ is hydrogen, hydroxy, halogen, alkyl having from 1 to 6 carbon atoms, alkoxy having from 1 to 6 carbon atoms, hydroxyalkyl having from 1 to 6 carbon atoms, or carboxylic acyloxy having from 2 to 6 carbon atoms;

$R^1_b$ is alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 3- or 4-pyridyl, 2-,4- or 5-pyrimidyl, 2- or 3-thienyl, 2- or 3-furanyl, carboxylic acyl having from 2 to 6 carbon atoms alkyl having from 1 to 10 carbon atoms which may be substituted with -COOH, hydroxy, halogen, alkoxy having from 1 to 6 carbon atoms, phenyl, 2-, 3- or 4-pyridyl, 2-, 4- or 5-pyrimidyl, 2- or 3-thienyl, 2- or 3-furanyl, carboxylic acyl having from 2 to 6 carbon atoms, cycloalkyl having from 3 to 7 carbon atoms or carboxylic acyloxy having from 1 to 6 carbon atoms;

$R^2_b$ is hydrogen, carboxylic acyl having from 1 to 6 carbon atoms, alkenyl having from 3 to 8 carbon atoms, alkynyl having 3 to 8 carbon atoms, alkyl having from 1 to 6 carbon atoms, $R^3_bR^4_bN(CH_2)_{nb}$- (wherein $R^3_b$ and $R^4_b$ are hydrogen, alkyl having from 1 to 6 carbon atoms or may be joined to complete a piperidine, morpholine, piperazine or pyrrolidine ring and nb is an integer of from 2 to 6), hydroxyalkyl having from 2 to 6 carbon atoms, dihydroxyalkyl having from 2 to 6 carbon atoms, hydroxyalkoxyalkyl having from 2 to 8 carbon atoms, or a cation derived from a pharmaceutically acceptable metal or an amine;

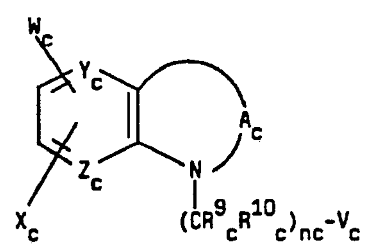

$I_c$

or a solvate thereof, wherein:

$A_c$ is

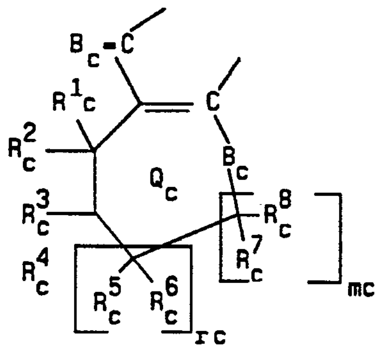

or

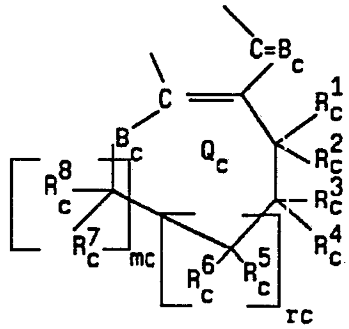

A'c A"c

$B_c$ is independently oxygen or sulfur;

$R^1{}_c$-$R^8{}_c$ may be the same or different and are hydrogen or alkyl having from 1 to 6 carbon atoms or two adjacent $R^1{}_c$-$R^8{}_c$ substituents may be combined to form an additional carbon to carbon bond;

rc and mc may be the same or different and are 0 or 1;

the ring labeled $Q_c$ may or optionally contain up to two additional double bonds;

nc is 0, 1 or 2;

$W_c$ and $X_c$ may be the same or different and are hydrogen, hydroxy, alkyl having from 1 to 6 carbon atoms, halogen, nitro, alkoxy having from 1 to 6 carbon atoms, tri-fluoromethyl, cyano, cycloalkyl having from 3 to 7 carbon atoms, alkenyloxy having from 3 to 6 carbon atoms, alkynyloxy having from 3 to 6 carbon atoms, $S(O)_{pc}$-$R^{11}{}_c$ {wherein pc is 0, 1 or 2 and $R^{11}{}_c$ is alkyl having from 1 to 6 carbon atoms}, $NHSO_2R^{11}{}_c$ {wherein $R^{11}{}_c$ is as defined above}, $NHSO_2CF_3$, $NHCOCF_3$, $SO_2NH_2$, $COR^{12}{}_c$ {wherein $R^{12}{}_c$ is OH, $NH_2$ or $OR^{11}{}_c$ (wherein $R^{11}{}_c$ is as defined above)}, O-$D_c$-$COR^{12}{}_c$ {wherein $D_c$ is alkylene having from 1 to 4 carbon atoms and $R^{12}{}_c$ is as defined above}, or $NHCOR^{13}{}_c$ {wherein $R^{13}{}_c$ is hydrogen, alkyl having from 1 to 6 carbon atoms, alkoxy having from 1 to 6 carbon atoms, $COR^{14}{}_c$ (wherein $R^{14}{}_c$ is hydroxy or alkoxy having from 1 to 6 carbon atoms) or $NHR^{15}{}_c$ (wherein $R^{15}{}_c$ is hydrogen or alkyl having from 1 to 6 carbon atoms}, or phenoxy {wherein the benzene ring may be substituted with any of the other substituents $W_c$ and $X_c$};

$Y_c$ and $Z_c$ may be the same or different and are CH or N;

$V_c$ is phenyl, naphthyl, indenyl, indanyl, 2-, 3- or 4-pyridyl, 2-, 3- or 5-pyrimidyl, 2- or 3-thienyl, 2- or 3-furyl or 2-, 4- or 5-thiazolyl, any of which may be substituted with $W_c$ and $X_c$ as defined above;

and $R^9{}_c$ and $R^{10}{}_c$ are independently hydrogen or alkyl having from 1 to 6 carbon atoms, for preparing a medicament for suppressing the immune response.

2. The use according to claim 1 wherein in formula $I_a$ $Y_a$ and $Z_a$ are both oxygen and/or
ra is zero and/or
$R^5{}_a$ and $R^6{}_a$ are both hydrogen and/or
qa is 2, 3 or 4 and/or
$R_a$ and $R'_a$ independently are hydrogen, methyl, ethyl, propyl, isopropyl, n-butyl or iso-butyl and/or
$A_a$ is phenyl or phenyl substituted with one or two substituted $Q_a$ and/or
$V_a$ is O, $S(O)_{na}$ or N-$R^8{}_a$ preferably O.

3. The use according to claim 1 or 2 wherein in formula $I_a$ all of a, b, c, and d are CH or
a and d are both N and b and c are both CH or
d is N and a, b and c are CH.

4. The use according to claim 1 wherein the compound of formula $I_a$ is

1'-phenyl-spiro[cyclopentane-1,3'-(1-8)naphthyridine]-2',4'-(1H)-dione;
1'-phenyl-spiro[cyclopentane-1,3'-quinoline]-2',4'-(1H)-dione;
1'-(4-methylphenyl)spiro[cyclopentane-1,3'-(1,8)naphthyridine]-2',4'-(1H)-dione;
1'-(4-chlorophenyl)spiro[cyclopentane-1,3'-(1,8)naphthyridine]-2',4'-(1H)-dione;
1'-(3,4-dichlorophenyl)spiro[cyclopentane-1,3'-(1,8)naphthyridine]-2',4'-(1H)-dione;
1'-(3-chlorophenyl)spiro[cyclopentane-1,3'-(1,8)naphthyridine]-2',4'-(1H)-dione;
1'-(3-methoxyphenyl)spiro[cyclopentane-1,3'-(1,8)naphthyridine]-2',4'-(1H)-dione;
1'-(3-hydroxyphenyl)spiro[cyclopentane-1,3'-(1,8)naphthyridine]-2,'4'-(1H)-dione;
1'-phenylspiro[cyclopentane-1,3'-quinoline]-2',4'-(1H)-dione;
1-phenyl-3',4,',5',6'-tetrahydrospiro[1,8-naphthyridine-3,2'-(2H)-pyran]-2,4-dione;
1-(3-methoxyyhenyl)-3',4',5',6'-tetrahydrospiro[1,8-naphthyridine-3,2'-(2H)pyran]-2,4-dione;

4,5-dihydro-1'-phenyl-spiro[furan-2(3H),3'(2'H) (1,8)naphthyridine]-2',4'(1'H)-dione;
1-phenyl-spiro[1,8-naphthyridine-3,2'-oxetane]-2,4-dione; or
1-(3-chlorphenyl)-3',4',5',6'-tetrahydrospiro[1,8-naphthyridine-3,2'-(2H)pyran]-2,4-dione or
solvate thereof.

5. The use according to claim 1 wherein in formula $I_b$ $X_b$ is CH and/or
wherein $Y_b$ is hydrogen, methoxy, trifluoromethyl or methylthio and/or $Z_b$ is hydrogen or methyl and/or
$R^1_b$ is n-alkyl having from 3 to 5 carbon atoms, alkenyl having from 3 to 4 carbon atoms, omegahydroxyalkyl having from 2 to 4 carbon atoms or omegacarboxylicacyloxyalkyl having from 6 to 9 carbon atoms and/or
$R^2_b$ is hydrogen, carboxylic acyl of from 2 to 4 carbon atoms, hydroxyalkyl of from 2 to 4 carbon atoms, $R^3_bR^4_bN(CH_2)_{nb}$- (in which $R^3_b$ and $R^4_b$ each independently represent hydrogen or alkyl of from 1 to 6 carbon atoms and nb represents an integer of from 2 to 6) or a cation derived from sodium, potassium, calcium, ethanolamine, N-methylglucamine, diethanolamine, ethylenediamine, tris(hydroxymethyl)aminomethane or lysine.

6. The use according to claim 1 wherein the compound of formula $I_b$ is 3-(n-butyl)-4-hydroxy-1-(3-methylthiophenyl)-1,8-naphthyridin-2(1H)-one,
4-acetoxy-1-phenyl-3-(2-propenyl)-1,8-naphthyridin-2(1H)-one,
3-(n-butyl)-4-(2-hydroxyethoxy)-1-phenyl-1,8-naphthyridin-2(1H)-one,
4-hydroxy-3-(2-propenyl)-1-phenyl-1,8-naphthyridin-2(1H)-one,
4-hydroxy-1-(3-methoxyphenyl)-3-(2-propenyl)-1,8-naphthyridin-2(1H)-one,
1-phenyl-4-propionyloxy-3-(4-propionyloxy-butyl)-1,8-naphthyridin-2(1H)-one, or
4-hydroxy-3-(2-hydroxyethyl)-1-phenyl-1,8-naphthyridin-2(1H)-one or
a compound of the formula

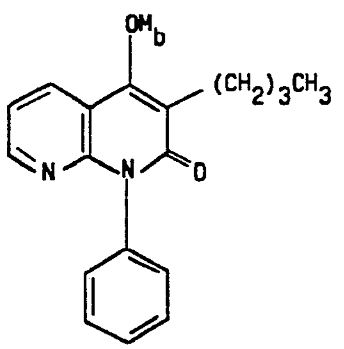

$II_b$

wherein $M_b$ is a cation derived from a pharmaceutically acceptable metal, an amine or amino acid, preferably being derived from sodium, potassium, calcium, ethanolamine, N-methylglucamine, diethanolamine, ethylenediamine, tris-(hydroxymethyl) aminomethane, or lysine.

7. The use according to claim 1 wherein in formula
$I_c$ nc is zero and/or
$Y_c$ is CH.

8. The use according to claim 1 wherein the compound of formula $I_c$ has the structural formula :

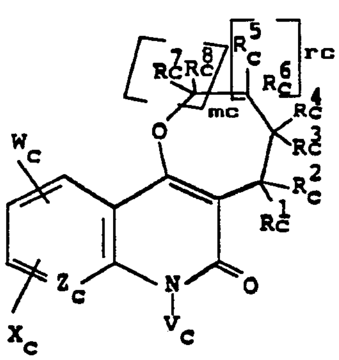

$II_c$

wherein $W_c$, $X_c$, $Z_c$, $V_c$, $R^1_c$ to $R^8_c$, rc and mc are as defined in claim 1
wherein preferably
$Z_c$ is N and/or

rc and mc are both zero or one or
the sum of rc and mc is one and/or
$B_c$ is oxygen and/or
$V_c$ is

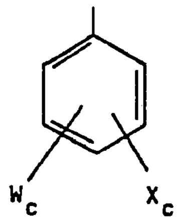

$IX_c$

9. The use according to claim 1 wherein the compound of formula $I_c$ has the structural formula:

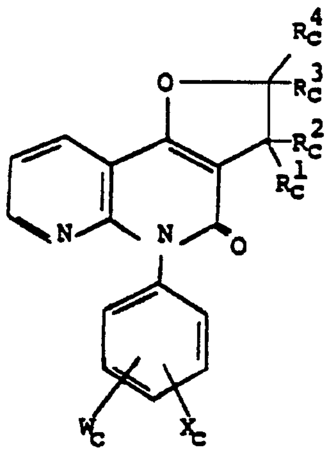

$III_c$

wherein $W_c$, $X_c$, $R^1{}_c$, $R^2{}_c$, $R^3{}_c$, and $R^4{}_c$ are as defined in claim 1, preferably $R^1{}_c$, $R^2{}_c$, $R^3{}_c$ and $R^4{}_c$ are hydrogen or methyl.

10. The use according to claim 1 wherein said compound of formula I has the structural formula:

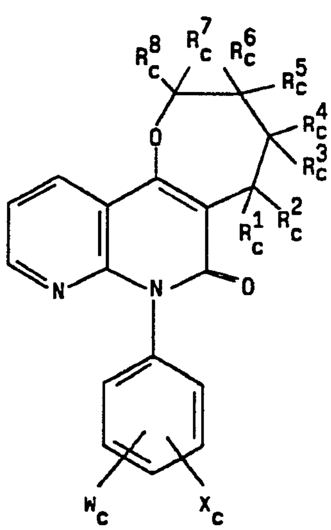

$IV_c$

wherein $W_c$, $X_c$ and $R^1{}_c$-$R^8{}_c$ are as defined in claim 1, preferably

$R^1_c$-$R^8_c$ are hydrogen or methyl, especially hydrogen, or one of $R^1_c$-$R^8_c$ is methyl and the rest are hydrogen and/or $W_c$ is 3-chloro and $X_c$ is hydrogen, chlorine or fluorine or $W_c$ is 3-methoxy and $X_c$ is hydrogen or fluorine or $W_c$ and $X_c$ are both hydrogen.

11. The use according to claim 1 wherein said compound of formula $I_c$ has the structural formula:

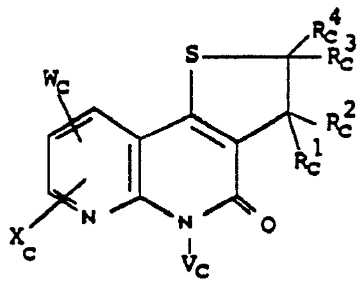

$VII_c$

wherein $W_c$, $X_c$, $V_c$, $R^1_c$, $R^2_c$, $R^3_c$ and $R^4_c$ are as defined in claim 1.

12. The use according to claim 1 wherein said compound has the structural formula:

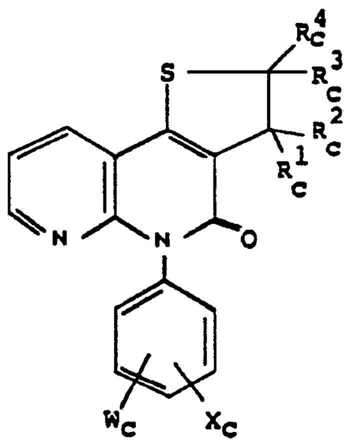

$VIII_c$

wherein $W_c$, $X_c$, $R^1_c$, $R^2_c$, $R^3_c$ and $R^4_c$ are as defined in claim 1, preferably. $W_c$ is 3-chloro and $X_c$ is hydrogen, chlorine or fluorine or $W_c$ is 3-methoxy and $X_c$ is hydrogen or fluorine or $W_c$ and $X_c$ are both hydrogen.

13. The use according to claim 1 wherein the compound of formula $I_c$ is
3,5-dihydro-5-phenyl-furo[3,2-c] [1,8]naphthyridin-4[2H]-one;
3,5-dihydro-5-phenyl-thieno[3,2-c] [1,8]-naphthyridin-4[2H]-one;
6-phenyl-2,3,4,6-tetrahydro-pyrano[3,2-c] [1,8]-naphthyridin-5-one;
2-methyl-3,5-dihydro-5-phenyl-furo[3,2-c] [1,8]-naphthyridin-4[2H]-one;
3,9-dihydro-9-phenyl-furo[2,3-b] [1,8]naphthyridin-4[2H]-one;
3,9-dihydro-9-(p-methylphenyl)-furo[2,3-b] [1,8]-naphthyridin-4[2H]-one;
3,9-dihydro-2-methyl-9-phenyl-furo[2,3-b] [1,8]-naphthyridin-4[2H]-one;
3,5-dihydro-5-(p-methylphenyl)-furo[3,2-c] [1,8]-naphthyridin-4[2H]-one;
3,5-dihydro-5-(p-fluorophenyl)-furo[3,2-c] [1,8]-naphthyridin-4[2H]-one;
3,5-dihydro-5-(m-methoxyphenyl)-furo[3,2-c] [1,8]-naphthyridin-4[2H]-one;
3,5-dihydro-5-(m-methylthiophenyl)-furo[3,2-c] [1,8]-naphthyridin-4[2H]-one;
3,9-dihydro-9-(p-fluorophenyl)-furo[2,3-b] [1,8]-naphthyridin-4[2H]-one;

3,9-dihydro-9-(m-methoxyphenyl)-furo[2,3-b] [1,8]naphthyridin-4[2H]-one;
3,9-dihydro-9-(m-methylthiophenyl)-furo[3,2-c] [1,8]-naphthyridin-4[2H]-one;
3,5-dihydro-5-(3,4-dichlorophenyl)-furo[3,2-c] [1,8]-naphthyridin-4[2H]-one;
3,5-dihydro-5-(3,4-dichlorophenyl)-2-methyl-furo [3,2-c] [1,8]-naphthyridin-4[2H]-one;
3,5-dihydro-5-(4-chlorophenyl)-furo[3,2-c] [1,8]-naphthyridin-4[2H]-one;
3,5-dihydro-5-(3-chlorophenyl)-furo[3,2-c] [1,8]-naphthyridin-4[2H]-one;
3,5-dihydro-5-(3-chlorophenyl)-2-methyl-furo[3,2-c]-[1,8]-naphthyridin-4[2H]-one;
3,5-dihydro-5-(4-fluorophenyl)-2-methyl-furo[3,2-c]-[1,8]-naphthyridin-4[2H]-one;
3,5-dihydro-5-(3-methoxyphenyl)-2-methyl-furo-[3,2-c] [1,8]-naphthyridin-4[2H]-one;
3,5-dihydro-5-(3,5-dichlorophenyl)-furo[3,2-c] [1,8]-naphthyridin-4[2H]-one;
3,5-dihydro-5-(3,5-dichlorophenyl)-2-methyl-furo[3,2-c] [1,8]-naphthyridin-4[2H]-one;
3,5-dihydro-5-phenyl-2,2-dimethyl-furo[3,2-c] [1,8]-naphthyridin-4[2H]-one;
3,5-dihydro-5-(3-methysulfonylaminophenyl)-2-methyl-furo[3,2-c] [1,8]-naphthyridin-4[2H]-one;
3,5-dihydro-5-(3-methylsulfonylaminophenyl)-furo[3,2-c] [1,8]-naphthyridin-4[2H]-one;
3,9-dihydro-9-(3,4-dichloronphenyl)-furo[2,3-b] [1,8]-naphthyridin-4[2H]-one;
3,9-dihydro-9-(4-chlorophenyl)-furo[2,3-b] [1,8]-naphthyridin-4[2H]-one;
3,9-dihydro-9-(3-chlorophenyl)-furo[2,3-b] [1,8]-naphthyridin-4[2H]-one;
3,9-dihydro-9-(3-chlorophenyl)-2-methyl-furo[2,3-b] [1,8]-naphthyridin-4[2H]-one;
3,9-dihydro-9-(4-fluorophenyl)-2-methyl-furo[2,3-b] [1,8]-naphthyridin-4[2H]-one;
3,9-dihydro-9-(3-methoxyphenyl)-2-methyl-furo[2,3-b] [1,8]-naphthyridin-4[2H]-one;
3,9-dihydro-9-(3, 5-dichlorophenyl)-furo[2,3-b] [1,8]-naphthyridin-4[2H]-one;
3,9-dihydro-9-(3,5-dichlorophenyl)-2-methyl-furo[2,3-b] [1,8]-naphthyridin-4[2H]-one;
3,9-dihydro-9-(3-methylsulfonylaminophenyl)-2-methyl-furo[2,3-b] [1,8]-naphthyridin-4[2H]-one;
6-(4-chlorophenyl)-2,3,4,6-tetrahydro-5H-pyrano[3,2-c]-[1,8]naphthyridin-5-one;
6-(3,4-dichlorophenyl)-2,3,4,6-tetrahydro-5H-pyrano [3,2-c] [1,8]naphthyridin-5-one;
6-(4-methoxyphenyl)-2,3,4,6-tetrahydro-5H-pyrano [3,2-c] [1,8]naphthyridin-5-one;
6-(4-methylphenyl)-2,3,4,6-tetrahydro-5H-pyrano [3,2-c] [1,8]naphthyridin-5-one;
10-(3,4-dichlorophenyl)-2,3,4,10-tetrahydro-5H-pyrano-[2,3-b] [1,8]naphthyridin-5-one;
10-(4-methoxyphenyl)-2,3,4,10-tetrahydro-5H-pyrano-[2,3-b] [1,8]naphthyridin-5-one;
10-(4-chlorophenyl)-2,3,4,10-tetrahydro-5H-pyrano-(2,3-b] [1,8] naphthyridin-5-one;
10-(4-methylphenyl)-2,3,4,10-tetrahydro-5H-pyrano-(2,3-b] [1,8]naphthyridin-5-one;
10-phenyl-2,3,4,10-tetrahydro-5H-pyrano-[2,3-b] [1,8]-naphthyridin-5-one;
7-phenyl-3,4,5,7-tetrahydro-oxepino[3,2-c] [1,8]-naphthyridin-6[2H]-one;
7-(4-chlorophenyl)-3,4,5,7-tetrahydro-oxepino-[3,2-c] [1,8]-naphthyridin-6[2H]-one;
7-(3-chlorophenyl)-3,4,5,7-tetrahydro-oxepino-[3,2-c] [1,8]-naphthyridin-6[2H]-one;
7-(3-methoxyphenyl)-3,4,5,7-tetrahydro-oxepino-[3,2-c] [1,8]-naphthyridin-6[2H]-one; or
7-(3-hydroxyphenyl)-3,4,5,7-tetrahydro-oxepino-[3,2-c] [1,8]-naphthyridin-6[2H]-one.

14. The use according to any one of claims 1 to 13 for preparing a medicament for oral use.

**Revendications**

1. Utilisation d'un azanaphtalène ayant la formule de structure $I_a$, $I_b$ ou $I_c$

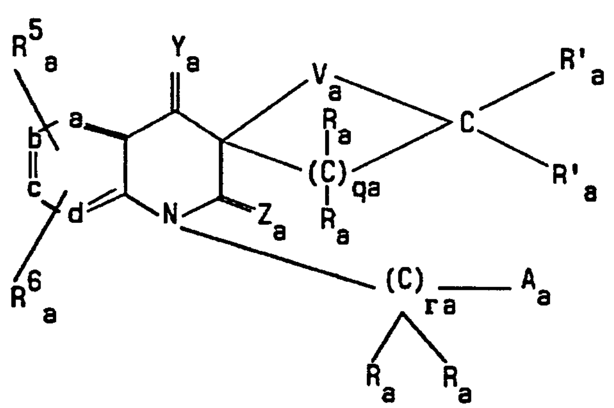

$I_a$

ou son solvat,
où:

deux des groupes de noyau a, b, c et d peuvent être CH ou N et les deux groupes restants représentent CH;

$Y_a$ et $Z_a$ représentent indépendamment O ou S;

$V_a$ représentent O, $S(O)_{na}$, N-$R^8{}_a$ ou 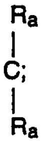;

chaque $R_a$ représente indépendamment un hydrogène, un alkyle ayant de 1 à 6 atomes de carbone, $CH_2OH$, $COR^7{}_a$ (où $R^7{}_a$ représente un hydrogène ou un alkyle ayant de 1 à 6 atomes de carbone) ou hydroxy, à condition qu'un seul groupe hydroxy puisse être attaché à un atome de carbone;

chaque $R'_a$ est indépendamment tel que défini pour $R_a$ ci-dessus à l'exception que lorsque $V_a$ représente O, $S(O)_{na}$ ou N-$R^8{}_a$, $R'_a$ ne peut être hydroxy;

$R^8{}_a$ est hydrogène, alkyle ayant de 1 6 atomes de carbone, acyle carboxylique ayant de 2 à 7 atomes de carbone, alkylsulfonyle ayant de 1 à 6 atomes de carbone, carboalcoxy ayant de 2 à 7 atomes de carbone, $CONH_2$, phényle ou pyridyle dont les deux derniers peuvent être substitués avec jusqu'à trois substituants $Q_a$, chaque $Q_a$ étant indépendamment hydroxy, alkyle ayant de 1 à 6 atomes de carbone, halogène, nitro, alcoxy ayant de 1 à 6 atomes de carbone, trifluorométhyle, cyano, cycloalkyle ayant de 3 à 7 atomes de carbone, alkényloxy ayant de 3 à 6 atomes de carbone, alkynyloxy ayant de 3 à 6 atomes de carbone, $S(O)_{na}$-$R^9{}_a$ {où na est tel que défini ci-dessus et $R^9{}_a$ est alkyle ayant de 1 à 6 atomes de carbone}, $NHSO_2R^9{}_a$ {où $R^9{}_a$ est tel que défini ci-dessus}, $NHSO_2CF_3$, $SO_2NH_2$, $COR^{10}{}_a$ {où $R^{10}{}_a$ est OH, $NH_2$ ou $OR^9{}_a$ (où $R^9{}_a$ est tel que défini ci-dessus)}, O-$B_a$-$COR^{10}{}_a$ {où $B_a$ est alkylène ayant de 1 à 4 atomes de carbone et $R^{10}{}_a$ est tel que défini ci-dessus}, ou $NHCOR^{11}{}_a$ {où $R^{11}{}_a$ est hydrogène, alkyle ayant de 1 à 6 atomes de carbone, alcoxy ayant de 1 à 6 atomes de carbone, $COR^{12}{}_a$ (où $R^{12}{}_a$ est hydroxy ou alcoxy ayant de 1 à 6 atomes de carbone) ou $NHR^{13}{}_a$ (où $R^{13}{}_a$ est hydrogène ou alkyle ayant de 1 à 6 atomes de carbone)};

$R^5{}_a$ et $R^6{}_a$ peuvent être identiques ou différents et sont hydrogène, alkyle ayant de 1 à 6 atomes de carbone, halogène, nitro, alcoxy ayant de 1 à 6 atomes de carbone, trifluorométhyle, alkylthio ayant de 1 à 6 atomes de carbone ou cyano;

na est 0, 1 ou 2;

ra est 0, 1 ou 2;

qa est un nombre entier de 1 à 5; et

$A_a$ est phényle, naphtyle, indényle, indanyle, 2-, 3- ou 4-pyridyle, 2-, 4-, 5- ou 6-pyrimidyle, 2- ou 3-pyrazinyle, 2- ou 3-furyle, 2- ou 3-thiényle, 2-, 4- ou 5-imidazolyle, 2-, 4- ou 5-thiazolyle ou 2-, 4- ou 5-oxazolyle, chacun d'entre eux pouvant être substitué avec trois substituants $Q_a$ tels que définis précédemment;

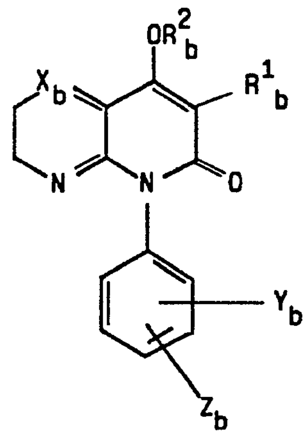

$I_b$

ou son solvat, où

$X_b$ est CH ou N;

$Y_b$ est hydrogène, hydroxy, benzyloxy, amino, sulfamyle, halogène, nitro, alkyle ayant de 1 à 6 atomes de carbone, alcoxy ayant de 1 à 6 atomes de carbone, acyle carboxylique ayant de 2 à 6 atomes de carbone, alkyle $S(O)_{mb}$ ayant de 1 à 6 atomes de carbone où mb est 0, 1 ou 2, trifluorométhyle, trifluorométhylthio, ou $COOA_b$ où $A_b$ est hydrogène, alkyle ayant de 1 à 6 atomes de carbone ou un cation dérivé d'un métal acceptable en pharmacie ou d'une amine;

$Z_b$ est hydrogène, hydroxy, halogène, alkyle ayant de 1 à 6 atomes de carbone, alcoxy ayant de 1 à 6 atomes de carbone, hydroxyalkyle ayant de 1 à 6 atomes de carbone ou acyloxy carboxylique ayant de 2 à 6 atomes de carbone;

$R^1{}_b$ est alkényle ayant de 2 à 10 atomes de carbone, alkynyle ayant de 2 à 10 atomes de carbone, cycloalkyle ayant de 3 à 7 atomes de carbone, cycloalkényle ayant de 5 à 8 atomes de carbone, 2-, 3- ou 4-pyridyle, 2-, 4- ou 5- pyrimidyle, 2- ou 3-thiényle, 2- ou 3-furanyle, acyle carboxylique ayant de 2 à 6 atomes de carbone ou alkyle ayant de 1 à 10 atomes de carbone qui peut être substitué par -COOH, hydroxy, halogène, alcoxy ayant de 1 à 6 atomes de carbone, phényle, 2-, 3- ou 4-pyridyle, 2-, 4- ou 5-pyrimidyle, 2- ou 3-thiényle, 2- ou 3-furanyle, acyle carboxylique ayant de 2 à 6 atomes de carbone, cycloalkyle ayant de 3 à 7 atomes de carbone ou acyloxy carboxylique ayant de 1 à 6 atomes de carbone;

$R^2_b$ est hydrogène, acyle carboxylique ayant de 1 à 6 atomes de carbone, alkényle ayant de 3 à 8 atomes de carbone, alkynyle ayant de 3 à 8 atomes de carbone, alkyle ayant de 1 à 6 atomes de carbone, $R^3_bR^4_bN(CH_2)_{nb}$- (où $R^3_b$ et $R^4_b$ sont hydrogène, alkyle ayant de 1 à 6 atomes de carbone ou peuvent être joints pour compléter un noyau de pipéridine, morpholine, pipérazine ou pyrrolidine et nb est un nombre entier de 2 à 6), hydroxyalkyle ayant de 2 à 6 atomes de carbone, dihydroxyalkyle ayant de 2 à 6 atomes de carbone, hydroxyalcoxyalkyle ayant de 2 à 8 atomes de carbone ou un cation dérivé d'un sel acceptable en pharmacie ou d'une amine;

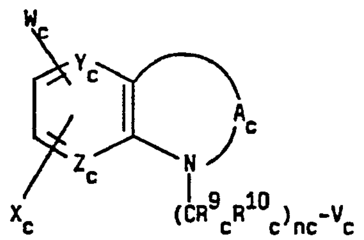

$I_c$

ou son solvat, où
$A_c$ est

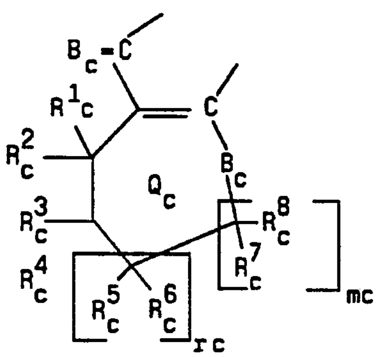

ou

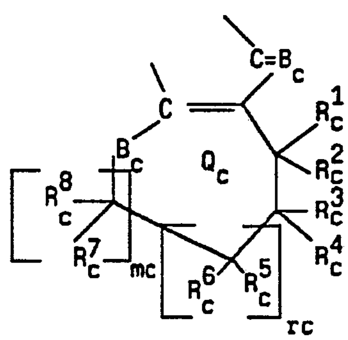

$A'_c$ $A''_c$

$B_c$ est indépendamment oxygène ou soufre;

$R^1_c$-$R^8_c$ peuvent être identiques ou différents et sont hydrogène ou alkyle ayant de 1 à 6 atomes de carbone ou bien deux substituants $R^1_c$-$R^8_c$ adjacents peuvent être combinés pour former une liaison additionnelle carbone à carbone;

rc et mc peuvent être identiques ou différents et sont 0 ou 1;

le noyau marqué $Q_c$ peut facultativement contenir jusqu'à deux doubles liaisons additionnelles;

nc est 0, 1 ou 2;

$W_c$ et $X_c$ peuvent être identiques ou différents et sont hydrogène, hydroxy, alkyle ayant de 1 à 6 atomes de carbone, halogène, nitro, alcoxy ayant de 1 à 6 atomes de carbone, trifluorométhyle, cyano, cycloalkyle ayant de 3 à 7 atomes de carbone, alkényloxy ayant de 3 à 6 atomes de carbone, alkynyloxy ayant de 3 à 6 atomes de carbone, $S(O)_{pc}$-$R^{11}_c$ {où pc est 0, 1 ou 2 et $R^{11}_c$ est alkyle ayant de 1 à 6 atomes de carbone}, $NHSO_2R^{11}_c$ {où $R^{11}_c$ est tel que défini ci-dessus}, $NHSO_2CF_3$, $NHCOCF_3$, $SO_2NH_2$, $COR^{12}_c$ {où $R^{12}_c$ est OH, $NH_2$ ou $OR^{11}_c$ (où $R^{11}_c$ est tel que défini ci-dessus)}, O-$D_c$-$COR^{12}_c$ {ou $D_c$ est alkylène ayant de 1 à 4 atomes de carbone et $R^{12}_c$ est tel que défini ci-dessus} ou $NHCOR^{13}_c$ {où $R^{13}_c$ est hydrogène, alkyle ayant de 1 à 6 atomes de carbone, alcoxy ayant de 1 à 6 atomes de carbone, $COR^{14}_c$ (où $R^{14}_c$ est hydroxy ou alcoxy ayant de 1 à 6 atomes de carbone) ou $NHR^{15}_c$ (où $R^{15}_c$ est hydrogène ou alkyle ayant de 1 à 6 atomes de carbone), ou phénoxy {où le noyau benzène peut être substitué par l'un ou l'autre des substituants $W_c$ et $X_c$)};

$Y_c$ et $Z_c$ peuvent être identiques ou différents et sont CH ou N;

$V_c$ est phényle, naphtyle, indényle, indanyle, 2-, 3- ou 4-pyridyle, 2-, 3- ou 5-pyrimidinyle, 2- ou 3-thiényle, 2- ou 3-furyle ou 2-, 4- ou 5-thiazolyle, chacun d'entre eux pouvant être substitué par $W_c$ et $X_c$ tels que définis ci-dessus; et

$R^9_c$ et $R^{10}_c$ sont indépendamment hydrogène ou alkyle ayant de 1 à 6 atomes de carbone, pour préparer un médicament pour supprimer la réponse immunitaire.

2. Utilisation selon la revendication 1 où dans la formule $I_a$, $Y_a$ et $Z_a$ sont tous deux oxygène et/ou
ra est O et/ou
$R^5{}_a$ et $R^6{}_a$ sont tous deux hydrogène et/ou
qa est 2, 3 ou 4 et/ou
$R_a$ et $R'_a$ sont indépendamment hydrogène, méthyle, éthyle, propyle, isopropyle, n-butyle ou isobutyle et/ou
$A_a$ est phényle ou phényle substitué par un ou deux substituants $Q_A$ et/ou
$V_a$ est O, $S(O)_{na}$ ou $N\text{-}R^8{}_a$, de préférence O.

3. Utilisation selon la revendication 1 ou 2 où dans la formule $I_a$, la totalité de a, b, c et d est CH ou
a et d sont tous deux N et b et c sont tous deux CH ou
d est N et a, b et c sont CH.

4. Utilisation selon la revendication 1 où le composé de formule $I_a$ est
1'-phényl-spiro [cyclopentane-1,3'-(1-8)-naphtyridine] -2',4'-(1'H)-dione ;
1'-phényl-spiro [cyclopentane-1,3'-quinoline] -2',4'-(1'H)-dione;
1'-(4-méthylphényl)spiro [cyclopentane-1,3'-(1,8)naphtyridine] -2',4'-(1'H)-dione;
1'-(4-chlorophényl)spiro [cyclopentane-1,3'-(1,8)naphtyridine] -2',4'-(1'H)-dione;
1'-(3,4-dichlorophényl)spiro [cyclopentane-1,3'-(1,8)naphtyridine] -2',4'-(1'H)-dione;
1'-(3-chlorophényl)spiro [cyclopentane-1,3'-(1,8)naphtyridine] -2',4'-(1'H)-dione;
1'-(3-méthoxyphényl)spiro [cyclopentane-1,3'-(1,8)naphtyridine] 2',4'-(1'H)-dione;
1'-(3-hydroxyphényl)spiro [cyclopentane-1,3'-(1,8)naphtyridine] -2',4'-(1'H)-dione;
1'-phénylspiro [cyclopentane-1,3'-quinoline]-2',4'-(1'H)-dione;
1-phényl-3',4',5',6'-tétrahydrospiro [1,8-naphtyridine-3,2'-(2H)pyran] -2,4-dione;
1-(3-méthoxyphényl)-3',4',5',6'-tétrahydrospiro-[1,8-naphtyridine-3,2'-(2H)pyran]-2,4-dione;
4,5-dihydro-1'-phényl-spiro [furan - 2(3H),3'(2'H)-(1,8)naphtyridine] -2',4'(1'H)-dione;
1-phényl-spiro [1,8-naphtyridine-3,2'-oxétane] -2,4-dione; ou
1-(3-chlorophényl)-3',4',5',6'-tétrahydrospiro-[1,8-naphtyridine-3,2'-(2H)pyran] -2,4-dione ou leur solvat.

5. Utilisation selon la revendication 1 où dans la formule $I_b$, $X_B$ est CH et/ou
où $Y_b$ est hydrogène, méthoxy, trifluorométhyle ou méthylthio et/ou $Z_b$ est hydrogène ou méthyle et/ou
$R^1{}_b$ est n-alkyle ayant de 3 à 5 atomes de carbone, alkényle ayant de 3 à 4 atomes de carbone, omégahydroxyalkyle ayant de 2 à 4 atomes de carbone ou omégacarboxylacyloxyalkyle ayant de 6 à 9 atomes de carbone et/ou
$R^2{}_b$ est hydrogène, acyle carboxylique de 2 à 4 atomes de carbone, hydroxyalkyle de 2 à 4 atomes de carbone, $R^3{}_bR^4{}_bN(CH_2)_{nb}$- (où $R^3{}_b$ et $R^4{}_b$ représentent chacun indépendamment hydrogène ou alkyle de 1 à 6 atomes de carbone et nb représente un nombre entier de 2 à 6) ou un cation dérivé du sodium, du potassium, du calcium, de l'éthanolamine, de la n-méthylglucamine, de la diéthanolamine, de l'éthylènediamine, du tris-(hydroxyméthyl) aminométhane ou de la lysine.

6. Utilisation selon la revendication 1 où le composé de formule $I_b$ est 3-(n-butyl)-4-hydroxy-1-(3-méthylthiophényl)-1,8-naphtyridin-2 (1H)-one,
4-acétoxy-1-phényl-3-(2-propényl)-1,8-naphtyridin-2(1H)-one,
3-(n-butyl)-4-(2-hydroxyéthoxy)-1-phényl-1,8-naphtyridin-2(1H)-one,
4-hydroxy-3-(2-prophényl)-1-phenyl-1,8-naphtyridin-2(1H)-one,
4-hydroxy-1-(3-méthoxyphényl)-3-(2-propényl)-1,8-naphtyridin-2(1H)-one,
1-phényl-4-propionyloxy-3-(4-propionyloxy-butyl)-1,8-naphtyridin-2(1H)-one, ou
4-hydroxy-3-(2-hydroxyéthyl)-phényl-1,8-naphtyridin-2(1H)-one ou
un composé de la formule

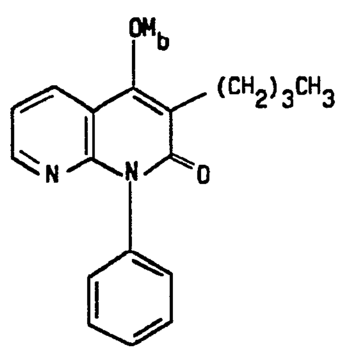

IIb

où $M_b$ est un cation dérivé d'un métal acceptable en pharmacie, d'une amine ou d'un acide aminé, étant de préférence dérivé du sodium, potassium, calcium, éthanolamine, n-méthylglucamine, diéthanolamine, éthylènediamine, tris-(hydroxyméthyl)aminométhane ou lysine.

7. Utilisation selon la revendication 1 où dans la formule $I_c$, nc est zéro et/ou $Y_c$ est CH.

8. Utilisation selon la revendication 1 où le composé de formule $I_c$ a la formule de structure:

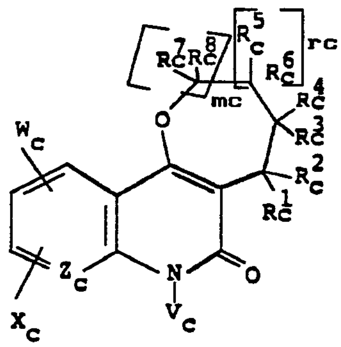

$II_c$

où $W_c$, $X_c$, $Z_c$, $V_c$, $R^1{}_c$ à $R^8{}_c$, rc et mc sont tels que définis à la revendication 1
où de préférence
$Z_c$ est N et/ou
rc et mc sont tous deux zéro ou un ou la somme de rc et mc est un et/ou
$B_c$ est oxygène et/ou
$V_c$ est

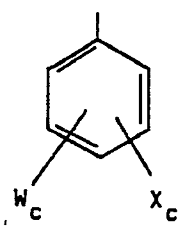

$IX_c$

9. Utilisation selon la revendication 1 où le composé de formule $I_c$ a la formule de structure:

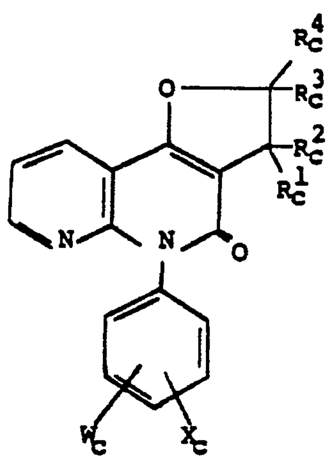

$III_c$

où $W_c$, $X_c$, $R_c$, $R^2{}_c$, $R^3{}_c$ et $R^4{}_c$ sont tels que définis à la revendication 1, de préférence $R^1{}_c$, $R^2{}_c$, $R^3{}_c$ et $R^4{}_c$ sont hydrogène ou méthyle.

10. Utilisation selon la revendication 1 où ledit composé de formule I a la formule de structure:

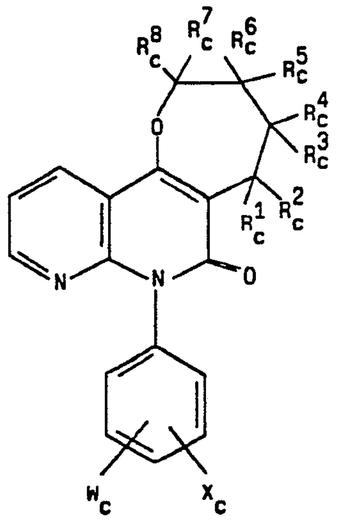

IVc

où $W_c$, $X_x$ et $R^1{}_c$-$R^8{}_c$ sont tels que définis à la revendication 1, de préférence $R^1{}_c$-$R^8{}_c$ sont hydrogène ou méthyle, en particulier hydrogène,
ou l'un de $R^1{}_c$-$R^8{}_c$ est méthyle et le reste sont hydrogène et/ou
$W_c$ est 3-chloro et $X_c$ est hydrogène, chlore ou fluor
ou
$W_c$ est 3-méthoxy et $X_c$ est hydrogène ou fluor ou
$W_c$ et $X_c$ sont tous deux hydrogène.

11. Utilisation selon la revendication 1 où ledit composé de formule $I_c$ a la formule de structure:

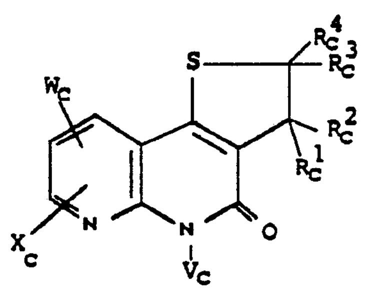

VIIc

où $W_c$, $X_c$, $V_c$, $R^1{}_c$, $R^3{}_c$ et $R^4{}_c$ sont tels que définis à la revendication 1.

12. Utilisation selon la revendication 1 où ledit composé a la formule de structure:

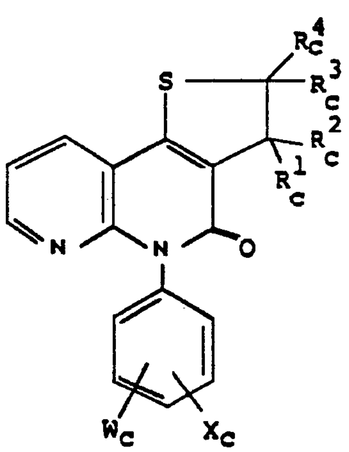

VIIIc

où $W_c$, $X_c$, $R^1_c$, $R^2_c$, $R^3_c$ et $R^4_c$ sont tels que définis à la revendication 1, de préférence
$W_c$ est 3-chloro et $X_c$ est hydrogène, chlore ou fluor
ou
$W_c$ est 3-méthoxy et $X_c$ est hydrogène ou fluor ou
$W_c$ et $X_c$ sont tuos deux de l'hydrogène.

13. Utilisation selon la revendication 1 où le composé de formule $I_c$ est
3,5-dihydro-5-phényl-furo[3,2-c] [1,8]-naphtyridin-4 [2H]-one;
3,5-dihydro-5-phényl-thiéno[3,2-c] [1,8]-naphtyridin-4 [2H-one;
6-phényl-2,3,4,6-tétrahydro-pyrano [3,2-c [1,8]-naphtyridin-5-one;
2-méthyl-3,5-dihydro-5-phényl-furo [3,2-c] [1,8]-naphtyridin-4[2H]-one;
3,9-dihydro-9-phényl-furo[2,3-b] [1,8]-naphtyridin-4 [2H]-one;
3,9-dihydro-9-(p-méthylphényl)-furo[2,3-b] [1,8]-naphtyridin-4 [2H]-one;
3,9-dihydro-2-méthyl-9-phényl-furo [2,3-b] [1,8]naphtyridin-4 [2H]-one;
3,5-dihydro-5-(p-méthylphényl)-furo [3,2-c [1,8]-naphtyridin-4 [2H]-one;
3,5-dihydro-5-(p-fluorophényl)-furo [3,2-c] [1,8]-naphtyridin-4 [2H]-one;
3,5-dihydro-5-(m-méthoxyphényl)-furo [3,2-c] [1,8]-naphtyridin-4 [2H]-one;
3,5-dihydro-5-(m-méthylthiophényl)-furo [3,2-c]-[1,8]-naphtyridin-4 [2H]-one;
3,9-dihydro-9-(p-fluorophényl)-furo [2,3-b] [1,8]-naphtyridin-4 [2H]-one;
3,9-dihydro-9-(m-méthoxyphényl)-furo[2,3-b]-[1,8]naphtyridin-4 [2H]-one;
3,9-dihydro-9-(m-méthylthiophényl) -furo [3,2-c]-[1,8]naphtyridin-4 [2H]-one;
3,5-dihydro-5-(3,4-dichlorophényl)-furo [3,2-c]-[1,8]-naphtyridin-4 [2H]-one;
3,5-dihydro-5-(3,4-dichlorophényl)-2-méthyl-furo [3,2-c] [1,8]-naphtyridin-4 [2H]-one;
3,5-dihydro-5-(4-chlorophényl)-furo [3,2-c]-[1,8]-naphtyridin-4 [2H]-one;
3,5-dihydro-5-(3-chlorophényl)-furo [3,2-c]-[1,8]-naphtyridin-4 [2H]-one;
3,5-dihydro-5-(3-chlorophényl)-2-méthyl-furo-[3,2-c] [1,8]-naphtyridin-4 [2H]-one;
3,5-dihydro-5-(4-fluorophényl)-2-méthyl-furo-[3,2-c] [1,8]-naphtyridin-4 [2H]-one;
3,5-dihydro-5-(3-méthoxyphényl)-2-méthyl-furo-[3,2-c] [1,8]-naphtyridin-4 [2H]-one;
3,5-dihydro-5-(3,5-dichlorophényl)-furo [3,2-c] [1,8]-naphtyridin-4 [2H]-one;
3,5-dihydro-5-(3,5-dichlorophényl)-2-méthyl-furo [3,2-c] [1,8]-naphtyridin-4 [2H]-one;
3,5-dihydro-5-phényl-2,2-diméthyl-furo [3,2 -c]-[1,8]-naphtyridin-4 [2H]-one;
3,5-dihydro-5-(3-méthylsulfonylaminophényl)-2-méthyl-furo [3,2-c] [1,8]-naphtyridin-4 [2H]-one;
3,5-dihydro-5-(3-méthylsulfonylaminophényl)-furo [3,2-c] [1,8]-naphtyridin-4 [2H]-one;
3,9-dihydro-9-(3,4-dichlorophényl)-furo [2,3-b]-[1,8]-naphtyridin-4 [2H]-one;
3,9-dihydro-9-(4-chlorophényl)-furo[2,3-b]-[1,8]-naphtyridin-4[2H]-one;
3,9-dihydro-9-(3-chlorophényl)-furo[2,3-b]-[1,8]-naphtyridin-4[2H]-one;
3,9-dihydro-9-(3-chlorophényl)-2-méthyl-furo-[2,3-b] [1,8]-naphtyridin-4 [2H]-one;
3,9-dihydro-9-(4-fluorophényl)-2-méthyl-furo-[2,3-b] [1,8]-naphtyridin-4 [2H]-one;
3,9-dihydro-9-(3-méthoxyphényl)-2-méthyl-furo-[2,3-b] [1,8]-naphtyridin-4 [2H]-one;
3,9-dihydro-9-(3,5-dichlorophényl)-furo[2,3-b]-[1,8]-naphtyridin-4[2H]-one;
3,9-dihydro-9-(3,5-dichlorophényl)-2-méthyl-furo [2,3-b] [1,8] naphtyridin-4 [2H]-one;
3,9-dihydro-9-(3-méthylsulfonylaminophényl)-2-méthyl-furo [2,3-b] [1,8]-naphtyridin-4 [2H]-one;
6-(4-chlorophényl)-2,3,4,6-tétrahydro-5H-pyrano [3,2-c] [1,8] naphtyridin-5-one;
6-(3,4-dichlorophényl)-2,3,4,6-tétrahydro-5H-pyrano [3,2-c] [1,8] naphtyridin-5-one;
6-(4-méthoxyphényl)-2,3,4,6-tétrahydro-5H-pyrano [3,2-c] [1,8]-naphtyridin-5-one;
6-(4-méthylphényl)-2,3,4,6-tétrahydro-5H-pyrano-[3,2-c] [1,8] naphtyridin-5-one;
10-(3,4-dichlorophényl)-2,3,4,10-tétrahydro-5H-pyrano-[2,3-b] [1,8] naphtyridin-5-one;
10-(4-méthoxyphényl)-2,3,4,10-tétrahydro-5H-pyrano [2,3-b] [1,8] naphtyridin-5-one;
10-(4-chlorophényl)-2,3,4,10-tétrahydro-5H-pyrano [2,3-b] [1,8] naphtyridin-5-one;
10-(4-méthylphényl)-2,3,4,10-tétrahydro-5H-pyrano [2,3-b] [1,8] naphtyridin-5-one;
10-phényl-2,3,4,10-tétrahydro-5H-pyrano- [2,3-b]- [1,8] -naphtyridin-5-one;
7-phényl-3,4,5,7-tétrahydro-oxépino [3,2-c]-[1,8]-naphtyridin-6 [2H]-one;
7-(4-chlorophényl)-3,4,5,7-tétrahydro-oxépino-[3,2-c]-[1,8]-naphtyridin-6[2H]-one;
7-(3-chlorophényl)-3,4,5,7-tétrahydro-oxépino-[3,2-c] [1,8] -naphtyridin-6 [2H]-one;
7-(3-méthoxyphényl)-3,4,5,7-tétrahydro-oxépino-[3,2-c] [1,8]-naphtyridin-6 [2H]-one; ou
7-(3-hydroxyphényl)-3,4,5,7-tétrahydro-oxépino-[3,2-c] [1,8]-naphtyridin-6 [2H]-one.

14. Utilisation selon l'une quelconque des revendications 1 à 13 pour la préparation d'un médicament pour usage oral.

**Patentansprüche**

1. Verwendung eines Azanaphthalins mit der Strukturformel $I_a$, $I_b$ oder $I_c$

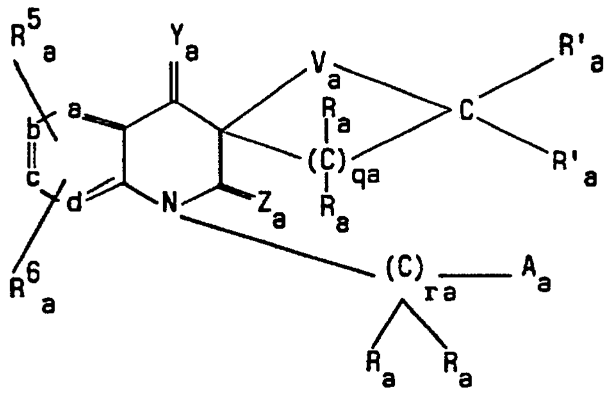

$I_a$

oder eines Solvats desselben, worin
zwei der Ring-Gruppen a, b, c und d CH oder N sein können und die verbleibenden zwei Gruppen CH darstellen;

$Y_a$ und $Z_a$ unabhängig voneinander O oder S darstellen;

$V_a$ O, $S(O)_{na}$ oder 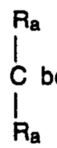 bezeichnet;

jedes $R_a$ unabhängig Wasserstoff, Alkyl mit 1 bis 6 Kohlenstoff-Atomen, $CH_2OH$, $COR^7_a$ (worin $R^7_a$ Wasserstoff oder Alkyl mit 1 bis 6 Kohlenstoff-Atomen bezeichnet) oder Hydroxy darstellt, mit der Maßgabe, daß nur eine Hydroxy-Gruppe an ein Kohlenstoff-Atom gebunden sein kann;

jedes $R'_a$ unabhängig die im Vorstehenden für $R_a$ angegebenen Bedeutungen haben kann, mit der Ausnahme, daß, wenn $V_a$ O, $S(O)_{na}$ oder $N\text{-}R^8_a$ ist $R'_a$ nicht Hydroxy sein kann;

$R^8_a$ Wasserstoff, Alkyl mit 1 bis 6 Kohlenstoff-Atomen, carboxylisches Acyl mit 2 bis 7 Kohlenstoff-Atomen, Alkylsulfonyl mit 1 bis 6 Kohlenstoff-Atomen, Carboalkoxy mit 2 bis 7 Kohlenstoff-Atomen, $CONH_2$, Phenyl oder Pyridyl ist, wovon die beiden letzten mit bis zu 3 Substituenten $Q_a$ substituiert sein können, wobei jedes $Q_a$ unabhängig Hydroxy, Alkyl mit 1 bis 6 Kohlenstoff-Atomen, Halogen, Nitro, Alkoxy mit 1 bis 6 Kohlenstoff-Atomen, Trifluoromethyl, Cyano, Cycloalkyl mit 3 bis 7 Kohlenstoff-Atomen, Alkenyloxy mit 3 bis 6 Kohlenstoff-Atomen, Alkinyloxy mit 3 bis 6 Kohlenstoff-Atomen, $S(O)_{na}\text{-}R^9_a$ (worin na die hierin angegebenen Bedeutungen hat und $R^9_a$ Alkyl mit 1 bis 6 Kohlenstoff-Atomen ist), $NHSO_2R^9_a$ (worin $R^9_a$ die im Vorstehenden angegebenen Bedeutungen hat), $NHSO_2CF_3$, $SO_2NH_2$, $COR^{10}_a$ {worin $R^{10}_a$ OH, $NH_2$ oder $OR^9_a$ ist (worin $R^9_a$ die im Vorstehenden angegebenen Bedeutungen hat}, $O\text{-}B_a\text{-}COR^{10}_a$ (worin $B_a$ Alkylen mit 1 bis 4 Kohlenstoff-Atomen ist und $R^{10}_a$ die im Vorstehenden angegebenen Bedeutungen hat) oder $NHCOR^{11}_a$ {worin $R^{11}_a$ Wasserstoff, Alkyl mit 1 bis 6 Kohlenstoff-Atomen, Alkoxy mit 1 bis 6 Kohlenstoff-Atomen, $COR^{12}_a$ (worin $R^{12}_a$ Hydroxy oder Alkoxy mit 1 bis 6 Kohlenstoff-Atomen ist) oder $NHR^{13}_a$ (worin $R^{13}_a$ Wasserstoff oder Alkyl mit 1 bis 6 Kohlenstoff-Atomen ist)} ist;

$R^5_a$ und $R^6_a$ gleich oder verschieden sein können und Wasserstoff, Alkyl mit 1 bis 6 Kohlenstoff-Atomen, Halogen, Nitro, Alkoxy mit 1 bis 6 Kohlenstoff-Atomen, Trifluoromethyl, Alkylthio mit 1 bis 6 Kohlenstoff-Atomen oder Cyano sind;

na 0, 1 oder 2 ist;

ra 0, 1 oder 2 ist;

qa eine ganze Zahl von 1 bis 5 ist; und

$A_a$ Phenyl, Naphthyl, Indenyl, Indanyl, 2-, 3- oder 4-Pyridyl, 2-, 4-, 5- oder 6-Pyrimidyl, 2- oder 3-Pyrazinyl, 2- oder 3-Furyl, 2- oder 3-Thienyl, 2-, 4- oder 5-Imidazolyl, 2-, 4- oder 5-Thiazolyl oder 2-, 4- oder 5-Oxazolyl, die sämtlich jeweils mit bis zu drei Substituenten $Q_a$, wie sie im Vorstehenden definiert sind, substituiert sein können;

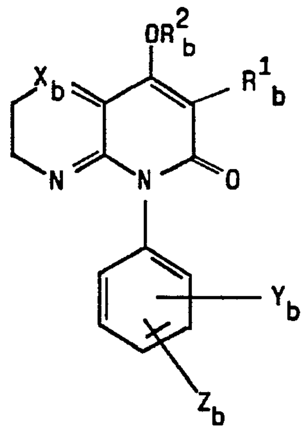

$I_b$

oder eines Solvats desselben, worin

$X_b$ CH oder N ist;

$Y_b$ Wasserstoff, Hydroxy, Benzyloxy, Amino, Sulfamyl, Halogen, Nitro, Alkyl mit 1 bis 6 Kohlenstoff-Atomen, Alkoxy mit 1 bis 6 Kohlenstoff-Atomen, carboxylisches Acyl mit 2 bis 6 Kohlenstoff-Atomen, Alkyl-$S(O)_{mb}$- mit 1 bis 6 Kohlenstoff-Atomen, worin mb 0, 1 oder 2 ist, Trifluoromethyl, Trifluoromethylthio oder $COOA_b$ ist, worin $A_b$ Wasserstoff, Alkyl mit 1 bis 6 Kohlenstoff-Atomen oder ein von einem pharmazeutisch annehmbaren Metall oder einem Amin abgeleitetes Kation ist;

$Z_b$ Wasserstoff, Hydroxy, Halogen, Alkyl mit 1 bis 6 Kohlenstoff-Atomen, Alkoxy mit 1 bis 6 Kohlenstoff-Atomen, Hydroxyalkyl mit 1 bis 6 Kohlenstoff-Atomen oder carboxylisches Acyloxy mit 2 bis 6 Kohlenstoff-Atomen ist;

$R^1_b$ Alkenyl mit 2 bis 10 Kohlenstoff-Atomen, Alkinyl mit 2 bis 10 Kohlenstoff-Atomen, Cycloalkyl mit 3 bis 7 Kohlenstoff-Atomen, Cycloalkenyl mit 5 bis 8 Kohlenstoff-Atomen, 2-, 3- oder 4-Pyridyl, 2-, 4- oder 5-Pyrimidyl, 2- oder 3-Thienyl, 2- oder 3-Furanyl, carboxylisches Acyl mit 2 bis 6 Kohlenstoff-Atomen oder Alkyl mit 1 bis 10 Kohlenstoff-Atomen ist, das durch -COOH, Hydroxy, Halogen, Alkoxy mit 1 bis 6 Kohlenstoff-Atomen, Phenyl, 2-, 3- oder 4-Pyridyl, 2-, 4- oder 5-Pyrimidyl, 2- oder 3-Thienyl, 2- oder 3-Furanyl, carboxylisches Acyl mit 2 bis 6 Kohlenstoff-Atomen, Cycloalkyl mit 3 bis 7 Kohlenstoff-Atomen oder carboxylisches Acyloxy mit 1 bis 6 Kohlenstoff-Atomen substituiert sein kann;

$R^2_b$ Wasserstoff, carboxylisches Acyl mit 1 bis 6 Kohlenstoff-Atomen, Alkenyl mit 3 bis 8 Kohlenstoff-Atomen, Alkinyl mit 3 bis 8 Kohlenstoff-Atomen, Alkyl mit 1 bis 6 Kohlenstoff-Atomen, $R^3_bR^4_bN(CH_2)_{nb}$- (worin $R^3_b$ und $R^4_b$ Wasserstoff, Alkyl mit 1 bis 6 Kohlenstoff-Atomen oder so miteinander verbunden sind, daß sie einen Piperidin-, Morpholin-, Piperazin- oder Pyrrolidin-Ring vervollständigen, und nb eine ganze Zahl von 2 bis 6 ist), Hydroxyalkyl mit 2 bis 6 Kohlenstoff-Atomen, Dihydroxyalkyl mit 2 bis 6 Kohlenstoff-Atomen, Hydroxyalkoxyalkyl mit 2 bis 8 Kohlenstoff-Atomen oder ein von einem pharmazeutisch annehmbaren Metall oder einem Amin abgeleitetes Kation ist;

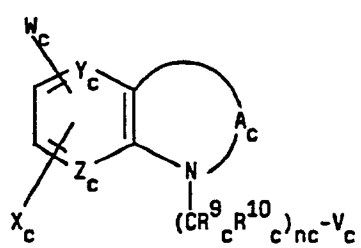

$I_c$

oder eines Solvates desselben, worin

$A_c$

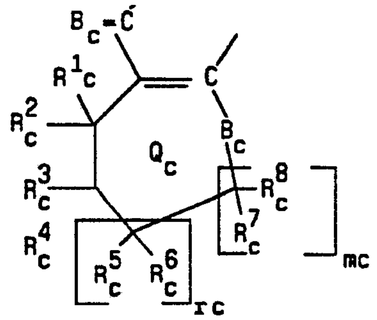

A'c

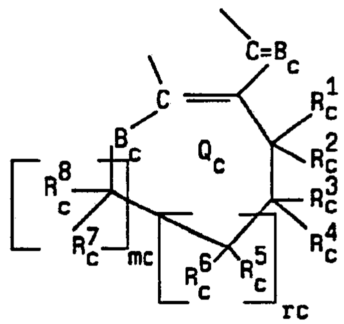

A"c

ist;

$B_c$ unabhängig Sauerstoff oder Schwefel ist;

$R^1_c$-$R^8_c$ gleich oder verschieden sein können und Wasserstoff oder Alkyl mit 1 bis 6 Kohlenstoff-Atomen sind oder zwei benachbarte $R^1_c$-$R^8_c$-Substiuenten so miteinander verbunden sein können, daß sie eine zusätzliche Kohlenstoff-Kohlenstoff-Bindung bilden;

rc und mc gleich oder verschieden sein können und 0 oder 1 sind;

der als $Q_c$ markierte Ring gegebenenfalls bis zu zwei zusätzliche Doppelbindungen enthalten kann;

nc 0, 1 oder 2 ist;

$W_c$ und $X_c$ gleich oder verschieden sein können und Wasserstoff, Hydroxy, Alkyl mit 1 bis 6 Kohlenstoff-Atomen, Halogen, Nitro, Alkoxy mit 1 bis 6 Kohlenstoff-Atomen, Trifluoromethyl, Cyano, Cycloalkyl mit 3 bis 7 Kohlenstoff-Atomen, Alkenyloxy mit 3 bis 6 Kohlenstoff-Atomen, Alkinyloxy mit 3 bis 6 Kohlenstoff-Atomen, $S(O)_{pc}$-$R^{11}_c$ {worin pc 0, 1 oder 2 ist und $R^{11}_c$ Alkyl mit 1 bis 6 Kohlenstoff-Atomen ist}, $NHSO_2R^{11}_c$ {worin $R^{11}_c$ die im Vorstehenden angegebenen Bedeutungen hat}, $NHSO_2CF_3$, $NHCOCF_3$, $SO_2NH_2$, $COR^{12}_c$ {worin $R^{12}_c$ OH, $NH_2$ oder $OR^{12}_c$ ist (worin $R^{11}_c$ die im Vorstehenden angegebenen Bedeutungen hat}, O-$D_c$-$COR^{12}_c$ {worin $D_c$ Alkylen mit 1 bis 4 Kohlenstoff-Atomen ist und $R^{12}_c$ die im Vorstehenden angegebenen Bedeutungen hat} oder $NHCOR^{13}_c$ {worin $R^{13}_c$ Wasserstoff, Alkyl mit 1 bis 6 Kohlenstoff-Atomen, Alkoxy mit 1 bis 6 Kohlenstoff-Atomen, $COR^{14}_c$ (worin Hydroxy oder Alkoxy mit 1 bis 6 Kohlenstoff-Atomen ist) oder $NHR^{15}_c$ (worin $R^{15}_c$ Wasserstoff oder Alkyl mit 1 bis 6 Kohlenstoff-Atomen ist)} oder Phenoxy {worin der Benzol-Ring mit irgendeinem der anderen Substituenten $W_c$ und $X_c$ substituiert sein kann} ist;

$Y_c$ und $Z_c$ gleich oder verschieden sein können und CH oder N sind;

$V_c$ Phenyl, Naphthyl, Indenyl, Indanyl, 2-, 3- oder 4-Pyridyl, 2-, 3- oder 5-Pyrimidyl, 2- oder 3-Thienyl, 2- oder 3-Furyl oder 2-, 4- oder 5-Thiazolyl ist, die sämtlich jeweils mit $W_c$ und $X_c$, wie sie im Vorstehenden definiert sind, substituiert sein können; und

$R^9_c$ und $R^{10}_c$ unabhängig Wasserstoff oder Alkyl mit 1 bis 6 Kohlenstoff-Atomen sind, für die Herstellung eines Medikaments zur Unterdrückung der Immunantwortreaktion.

2. Verwendung nach Anspruch 1, worin in der Formel $I_a$
$Y_a$ und $Z_a$ beide Sauerstoff sind und/oder
ra Null ist und/oder
$R^5_a$ und $R^6_a$ beide Wasserstoff sind und/oder
qa 2, 3 oder 4 ist und/oder
$R_a$ und $R'_a$ unabhängig Wasserstoff, Methyl, Ethyl, Propyl, Isopropyl, n-Butyl oder iso-Butyl sind und/oder
$A_a$ Phenyl oder mit ein oder zwei Substituenten $Q_a$ substituiertes Phenyl ist und/oder
$V_a$ O, $S(O)_{na}$ oder N-$R^8_a$, vorzugsweise O, ist.

3. Verwendung nach Anspruch 1 oder 2, worin in der Formel $I_a$
a, b, c und d sämtlich CH sind oder
a und d beide N sind und b und c beide CH sind oder
d N ist und a, b und c CH sind.

4. Verwendung nach Anspruch 1, worin die Verbindung der Formel $I_a$
1'-Phenyl-spiro [cyclopentan-1,3'-(1,8)naphthyridin] -2',4'-(1'H)-dion;
1'-Phenyl-spiro [cyclopentan-1,3'-chinolin] -2',4'-(1'H)-dion;
1'-(4-Methylphenyl)spiro [cyclopentan-1,3'-(1,8)naphthyridin] -2',4'-(1'H)-dion;
1'-(4-Chlorophenyl)spiro [cyclopentan-1,3'-(1,8)naphthyridin] -2',4'-(1'H)-dion;
1'-(3,4-Dichlorophenyl)spiro [cyclopentan-1,3'-(1,8)naphthyridin] -2',4'-(1'H)-dion;
1'-(3-Chlorophenyl)spiro [cyclopentan-1,3'-(1,8)naphthyridin] -2',4'-(1'H)-dion;
1'-(3-Methoxyphenyl)spiro [cyclopentan-1,3'-(1,8)naphthyridin] -2',4'-(1'H)-dion;
1'-(3-Hydroxyphenyl)spiro [cyclopentan-1,3'-(1,8)naphthyridin] -2',4'-(1'H)-dion;
1'-Phenyl-spiro [cyclopentan-1,3'-chinolin] -2',4'-(1'H)-dion;

1-Phenyl-3',4',5',6'-tetrahydrospiro[1,8-naphthyridin-3,2'-(2H)pyran] -2,4-dion;
1-(3-Methoxyphenyl)-3',4',5',6'-tetrahydrospiro[1,8-naphthyridin-3,2'-(2H)pyran] -2,4-dion;
4,5-Dihydro-1'-phenyl-spiro [furan-2(3H),3'(2'H)-(1,8)-naphthyridin] -2',4'-(1'H)-dion;
1'-Phenyl-spiro [1,8-naphthyridin-3,2'-oxetan] -2,4-dion;
oder
1-(3-Chlorophenyl)-3',4',5',6'-tetrahydrospiro[1,8-naphthyridin-3,2'-(2H)pyran]-2,4-dion
oder ein Solvat derselben ist.

5. Verwendung nach Anspruch 1, worin in der Formel $I_b$
$X_b$ CH ist und/oder worin
$Y_b$ Wasserstoff, Methoxy, Trifluoromethyl oder Methylthio ist und/oder
$Z_b$ Wasserstoff oder Methyl ist und/oder
$R^1_b$ n-Alkyl mit 3 bis 5 Kohlenstoff-Atomen, Alkenyl mit 3 bis 4 Kohlenstoff-Atomen, omega-Hydroxyalkyl mit 2 bis 4 Kohlenstoff-Atomen oder omega-carboxylisches Acyloxyalkyl mit 6 bis 9 Kohlenstoff-Atomen ist und/oder
$R^2_b$ Wasserstoff, carboxylisches Acyl mit 2 bis 4 Kohlenstoff-Atomen, Hydroxyalkyl mit 2 bis 4 Kohlenstoff-Atomen, $R^3_bR^4_bN(CH_2)_{nb}$- (worin $R^3_b$ und $R^4_b$ jeweils unabhängig Wasserstoff oder Alkyl mit 1 bis 6 Kohlenstoff-Atomen darstellen und nb eine ganze Zahl von 2 bis 6 bezeichnet) oder ein von Natrium, Kalium, Calcium, Ethanolamin, N-Methylglucamin, Diethanolamin, Ethylendiamin, Tris-(hydroxymethyl)aminomethan oder Lysin abgeleitetes Kation ist.

6. Verwendung nach Anspruch 1, worin die Verbindung der Formel $I_b$
3-(n-Butyl)-4-hydroxy-1-(3-methylthiophenyl)-1,8-naphthyridin-2(1H)-on,
4-Acetoxy-1-phenyl-3-(2-propenyl)-1,8-naphthyridin-2(1H)-on,
3-(n-Butyl)-4-(2-hydroxyethoxy)-1-phenyl-1,8-naphthyridin-2(1H)-on,
4-Hydroxy-3-(2-propenyl)-1-phenyl-1,8-naphthyridin-2(1H)-on,
4-Hydroxy-1-(3-methoxyphenyl)-3-(2-propenyl)-1,8-naphthyridin-2(1H)-on,
1-Phenyl-4-propionyloxy-3-(4-propionyloxybutyl)-1,8-naphthyridin-2(1H)-on oder
4-Hydroxy-3-(2-hydroxyethyl)-1-phenyl-1,8-naphthyridin-2(1H)-on oder
eine Verbindung der Formel

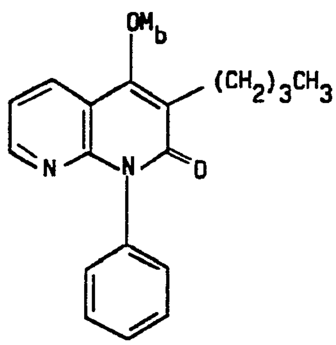

$II_b$

ist, worin
$M_b$ ein von einem pharmazeutisch annehmbaren Metall, einem Amin oder einer Aminosäure abgeleitetes Kation ist, das vorzugsweise von Natrium, Kalium, Calcium, Ethanolamin, N-Methylglucamin, Diethanolamin, Ethylendiamin, Tris-(hydroxymethyl)aminomethan oder Lysin abgeleitet ist.

7. Verwendung nach Anspruch 1, worin in der Formel $I_c$
nc Null ist und/oder
$Y_c$ CH ist.

8. Verwendung nach Anspruch 1, worin die Verbindung der Formel $I_c$ die Strukturformel

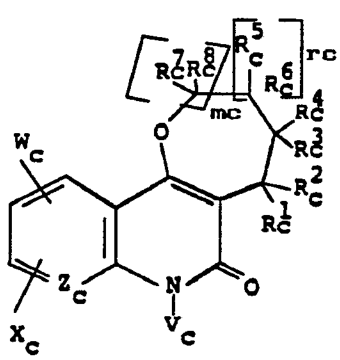

$II_c$

besitzt, worin $W_c$, $X_c$, $Z_c$, $V_c$, $R^1{}_c$ bis $R^8{}_c$, rc und mc die in Anspruch 1 angegebenen Bedeutungen haben, wobei vorzugsweise

$Z_c$ N ist und/oder

rc und mc beide Null oder Eins sind oder die Summe von rc und mc Eins ist und/oder

$B_c$ Sauerstoff ist und/oder

$V_c$

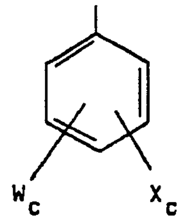

$IX_c$

ist.

9. Verwendung nach Anspruch 1, worin die Verbindung der Formel $I_c$ die Strukturformel

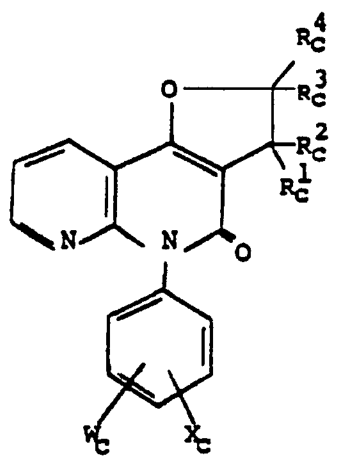

$III_c$

besitzt, worin $W_c$, $X_c$, $R^1{}_c$, $R^2{}_c$, $R^3{}_c$ und $R^4{}_c$ die in Anspruch 1 angegebenen Bedeutungen haben, wobei vorzugsweise $R^1{}_c$, $R^2{}_c$, $R^3{}_c$ und $R^4{}_c$ Wasserstoff oder Methyl sind.

10. Verwendung nach Anspruch 1, worin die Verbindung der Formel I die Strukturformel

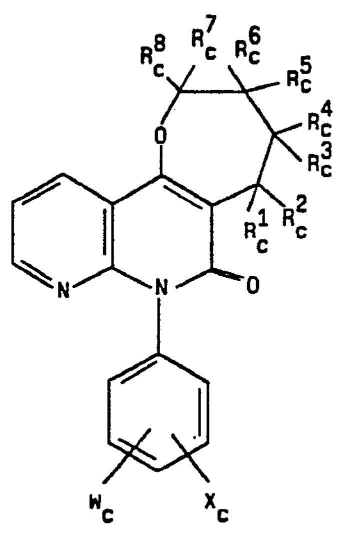

$IV_c$

besitzt, worin $W_c$, $X_c$ und $R^1{}_c$ bis $R^8{}_c$ die in Anspruch 1 angegebenen Bedeutungen haben, wobei vorzugsweise $R^1{}_c$ bis $R^8{}_c$ Wasserstoff oder Methyl sind, insbesondere Wasserstoff sind oder einer der Substituenten $R^1{}_c$ bis $R^8{}_c$ Methyl ist und der Rest Wasserstoff ist, und/oder
$W_c$ 3-Chloro ist und $X_c$ Wasserstoff, Chlor oder Fluor ist, oder $W_c$ 3-Methoxy ist und $X_c$ Wasserstoff oder Fluor ist, oder $W_c$ und $X_c$ beide Wasserstoff sind.

11. Verwendung nach Anspruch 1, worin die Verbindung der Formel $I_c$ die Strukturformel

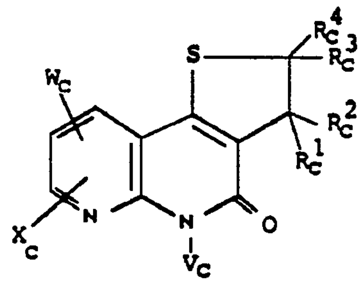

$VII_c$

besitzt, worin $W_c$, $X_c$, $V_c$, $R^1{}_c$, $R^2{}_c$, $R^3{}_c$ und $R^3{}_c$ die in Anspruch 1 angegebenen Bedeutungen haben.

12. Verwendung nach Anspruch 1, worin die genannte Verbindung die Strukturformel

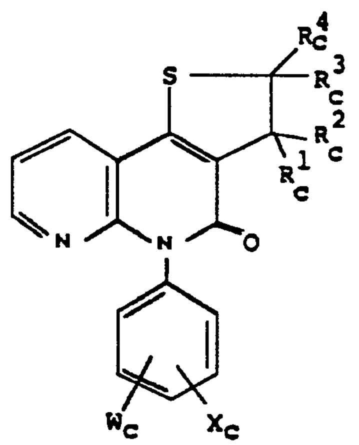

$VIII_c$

besitzt, worin $W_c$, $X_c$, $R^1{}_c$, $R^2{}_c$, $R^3{}_c$ und $R^4{}_c$ die in Anspruch 1 angegebenen Bedeutungen haben, wobei vorzugsweise $W_c$ 3-Chloro ist und $X_c$ Wasserstoff, Chlor oder Fluor ist, oder $W_c$ 3-Methoxy ist und $X_c$ Wasserstoff oder Fluor ist, oder $W_c$ und $X_c$ beide Wasserstoff sind.

13. Verwendung nach Anspruch 1, worin die Verbindung der Formel $I_c$
3,5-Dihydro-5-phenyl-furo[3,2-c] [1,8]naphthyridin-4[2H]-on;
3,5-Dihydro-5-phenyl-thieno[3,2-c] [1,8]naphthyridin-4[2H]-on;
6-Phenyl-2,3,4,6-tetrahydropyrano[3,2-c] [1,8]naphthyridin-5-on;
2-Methyl-3,5-dihydro-5-phenyl-furo[3,2-c] [1,8]naphthyridin-4[2H]-on;
3,9-Dihydro-9-phenyl-furo[2,3-b] [1,8]naphthyridin-4[2H]-on;
3,9-Dihydro-9-(p-Methylphenyl)-furo[2,3-b] [1,8]naphthyridin-4[2H]-on;
3,9-Dihydro-2-methyl-9-phenyl-furo[2,3-b] [1,8]naphthyridin-4[2H]-on;
3,5-Dihydro-5-(p-methylphenyl)-furo[3,2-c[1,8]naphthyridin-4[2H]-on;
3,5-Dihydro-5-(p-fluorophenyl)-furo[3,2-c] [1,8]naphthyridin-4[2H]-on;
3,5-Dihydro-5-(m-methoxyphenyl)-furo[3,2-c] [1,8]naphthyridin.4[2H]-on;
3,5-Dihydro-5-(m-methylthiophenyl)-furo[3,2-c] [1,8]-naphthyridin-4[2H]-on;
3,9-Dihydro-9-(p-fluorophenyl)-furo[2,3-b] [1,8]naphthyridin-4-[2H]-on;
3,9-Dihydro-9-(m-methoxyphenyl)-furo[2,3-b] [1,8]naphthyridin-4[2H]-on;
3,9-Dihydro-9-(m-methylthiophenyl)-furo[3,2-c] [1,8]-naphthyridin-4[2H]-on;
3,5-Dihydro-5-(3,4-dichlorophenyl)-furo[3,2-c] [1,8]-naphthyridin-4[2H]-on;
3,5-Dihydro-5-(3,4-dichlorophenyl)-2-methyl-furo[3,2-c] [1,8]naphthyridin[2H]-on;
3,5-Dihydro-5-(4-chlorophenyl)-furo[3,2-c] [1,8]naphthyridin-4[2H]-on;
3,5-Dihydro-5-(3-chlorophenyl)-furo[3,2] [1,8]naphthyridin-4[2H]-on;
3,5-Dihydro-5-(3-chlorophenyl)-2-methyl-furo[3,2-c] [1,8] naphthyridin-4[2H]-on;

3,5-Dihydro-5-(4-fluorphenyl)-2-methyl-furo[3,2-c] [1,8] naphthyridin-4[2H]-on;
3,5-Dihydro-5-(3-methoxyphenyl)-2-methyl-furo[3,2-c] naphthyridin-4[2H]-on;
3,5-Dihydro-5-(3,5-dichlorophenyl)-furo[3,2-c] [1,8]-naphthyridin-4[2H]-on;
3,5-Dihydro-5-(3,5-dichlorophenyl)-2-methyl-furo[3,2-c] [1,8] naphthyridin-4[2H]-on;
3,5-Dihydro-5-phenyl-2,2-dimethyl-furo[3,2-c] [1,8]naphthyridin-4[2H]-on;
3,5-Dihydro-5-(3-methylsulfonylaminophenyl)-2-methyl-furo [3,2-c] [1,8]naphthyridin-4[2H]-on;
3,5-Dihydro-5-(3-methylsulfonylaminophenyl)-furo [3,2-c] [1,8]naphthyridin-4[2H]-on;
3,9-Dihydro-9-(3,4-dichlorophenyl)-furo[2,3-b] [1,8]-naphthyridin-4[2H-on;
3,9-Dihydro-9-(4-chlorophenyl)-furo[2,3-b] [1,8]naphthyridin-4[2H]-on;
3,9-Dihydro-9-(3-chlorophenyl)-furo[2,3-b] [1,8]naphthyridin-4[2H]-on;
3,9-Dihydro-9-(3-chlorophenyl)-2-methyl-furo[2,3-b] [1,8] naphthyridin-4[2H]-on;
3,9-Dihydro-9-(4-fluorophenyl)-2-methyl-furo[2,3-b] [1,8] naphthyridin-4[2H]-on;
3,9-Dihydro-9-(3-methoxyphenyl)-2-methyl-furo[2,3-b] [1,8] naphthyridin-4[2H]-on;
3,9-Dihydro-9-(3,5-dichlorophenyl)-furo[2,3-b] [1,8]-naphthyridin-4[2H]-on;
3,9-Dihydro-9-(3,5-dichlorophenyl)-2-methyl-furo [2,3-b] [1,8] naphthyridin-4[2H]-on;
3,9-Dihydro-9-(3-methylsulfonylaminophenyl)-2-methyl-furo [2,3-b] [1,8]naphthyridin-4[2H]-on;
6-(4-Chlorophenyl)-2,3,4,6-tetrahydro-5H-pyrano [3,2-c] [1,8] naphthyridin-5-on;
6-(3,4-Dichlorophenyl)-2,3,4,6-tetrahydro-5H-pyrano [3,2-c] [1,8]naphthyridin-5-on;
6-(2-Methoxyphenol)-2,3,4,6-tetrahydro-5H-pyrano [3,2-c] [1,8]naphthyridin-5-on;
6-(4-Methylphenyl)-2,3,4,6-tetrahydro-5H-pyrano [3,2-c] [1,8]naphthyridin-5-on;
10-(3,4-Dichlorophenyl)-2,3,4,10-tetrahydro-5H-pyrano [2,3-b] [1,8]naphthyridin-5-on;
10-(4-Methyloxyphenyl)-2,3,4,10-tetrahydro-5H-pyrano [2,3-b [1,8]naphthyridin-5-on;
10-(4-Chlorophenyl)-2,3,4,10-tetrahydro-5H-pyrano [2,3-b] [1,8]naphthyridin-5-on;
10-(4-Methylphenyl)-2,3,4,10-tetrahydro-5H-pyrano [2,3-b] [1,8]naphthyridin-4-on;
10-Phenyl-2,3,4,10-tetrahydro-5H-pyrano[2,3-b] [1,8]-naphthyridin-5-on;
7-Phenyl-3,4,5,7-tetrahydro-oxepino[3,2-c] [1,8]naphthyridin-6[2H]-on;
7-(4-Chlorophenyl)-3,4,5,7-tetrahydro-oxepino[3,2-c] [1,8] naphthyridin-6[2H]-on;
7-(3-Chlorophenyl)-3,4,5,7-tetrahydro-oxepino[3,2-c] [1,8] naphthyridin-6[2H]-on;
7-(3-Methoxyphenyl)-3,4,5,7-tetrahydro-oxepino[3,2-c] [1,8] naphthyridin-6[2H]-on; oder
7-(3-Hydroxyphenyl)-3,4,5,7-tetrahydro-oxepino[3,2-c] [1,8] naphthyridin-6[2H]-on ist.

14. Verwendung nach irgendeinem der Ansprüche 1 bis 13 für die Herstellung eines Medikaments zur oralen Verwendung.